# FASCICULE DE BREVET EUROPEEN

(11) **EP 1 426 032 B1**
(45) Date de publication et mention de la délivrance du brevet: **16.11.2011**
(21) Numéro de dépôt: 03292935.8
(22) Date de dépôt: 26.11.2003
(51) Int. Cl.: A61K 8/42, A61K 8/87, A61K 8/34, A61Q 5/10

(54) **Composition de teinture d'oxydation pour fibres kératiniques comprenant un alcool gras non oxyalkyléné, un colorant d'oxydation, un polymère associatif et un amide d'une alcanolamine et d'un acide gras en C14-C30**
Zusammensetzung für die oxidative Färbung von keratinischen Fasern, enthaltend einen nicht alkoxylierten Fettalkohol, ein Oxidationsfärbemittel, ein assoziatives Polymer und ein Amid aus Alkanolamin und C14-C30 Fettsäure
Oxidative dyeing composition for keratinic fibres comprising a non oxyalkylenated fatty alcohol, an oxidation dye, an associative polymer and an amide from an alkanolamine and a C14-C30 fatty acid

(30) Priorité: 06.12.2002 FR 0215476
(43) Date de publication de la demande: 09.06.2004
(73) Titulaire: L'Oréal, 75008 Paris (FR)
(72) Inventeur: Cottard, Francois, 92400 Courbevoie (FR); Rondeau, Christine, 78500 Sartrouville (FR)
(74) Mandataire: Casalonga, Axel

(56) Documents cités:
- EP-A- 0 591 059
- EP-A- 1 142 555
- WO-A-01/68043
- WO-A-02/38115
- WO-A-02/45651
- WO-A-99/36047
- FR-A- 2 402 446

## Description

La présente invention est relative à une composition de teinture d'oxydation des fibres kératiniques, en particulier des fibres kératiniques humaines et plus particulièrement des cheveux, comprenant au moins un colorant d'oxydation, au moins un alcool gras non oxyalkyléné, au moins un polymère associatif et au moins un amide d'une alcanolamine et d'un acide gras en C₁₄-C₃₀·

Il est connu de teindre les fibres kératiniques, et en particulier les cheveux humains, avec des compositions de teinture contenant des précurseurs de colorants d'oxydation, généralement appelés « bases d'oxydation », en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des « bases d'oxydation » sur elles-mêmes, soit d'une condensation oxydative des « bases d'oxydation » sur des composés modificateurs de coloration, ou « coupleurs », qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les « bases d'oxydation » et d'autre part par les « coupleurs », permet l'obtention d'une palette très riche en coloris.

Les compositions qui contiennent des colorants d'oxydations et que l'on mélange avant emploi avec un oxydant se présentent souvent sous la forme de crèmes à base d'eau comprenant de manière classique des alcools gras et parfois des savons ou des polymères associatifs. Ces crèmes présentent une teneur importante en alcools gras, afin d'assurer la consistance et la stabilité du milieu.

Cependant, la demanderesse a constaté que ces compositions tinctoriales présentaient une évolution de la viscosité dans le temps, se traduisant par une dégradation de la facilité de mélange avec l'oxydant, et une dégradation des qualités d'usage.

De plus, les documents WO 01/45651, WO 01/68403 et EP 114255 décrivent des compositions tinctoriales à base de colorants d'oxydation, dialcools gras non oxyalkylénés, de polymères associatifs et d'acide d'alcanolamine et d'acides gras en C₁₄-C₃₀ > = <D>.

Or, après d'importantes recherches menées sur la question, la demanderesse vient maintenant de découvrir que des compositions de teinture d'oxydation comprenant un colorant d'oxydation, un alcool gras non oxyalkyléné, un polymère associatif choisi parmi les polymères amphiphiles et un amide d'une alcanolamine et d'un acide gras en C₁₄-C₃₀ le rapport en poids de l'amide d'un alcanolamine et d'un acide gras en C₁₄-C₃₀ sur le polymère associatif étant compris entre 5 et 20 présentent une consistance satisfaisante et une viscosité stable dans le temps, sans qu'il soit nécessaire d'augmenter la concentration en alcool gras.

La présente invention a ainsi pour objet une composition pour la teinture d'oxydation des fibres kératiniques, en particulier des fibres kératiniques humaines, telles que les cheveux, caractérisée en ce qu'elle comprend, dans un milieu approprié pour la teinture,
a) au moins un colorant d'oxydation,
b) au moins un alcool gras non oxyalkyléné,
c) au moins un polymère associatif, choisi parmi les polymères amphiphiles, et
d) au moins un amide d'une alcanolamine et d'un acide gras en C₁₄-C₃₀; le rapport en poids de l'amide d'un alcanolamine et d'un acide gras en C₁₄-C₃₀ sur le polymère associatif étant compris entre 5 et 20.

Un autre objet de l'invention porte sur une composition prête à l'emploi pour la teinture des fibres kératiniques qui comprend au moins un colorant d'oxydation, au moins un alcool gras non oxyalkyléné, au moins un polymère associatif, au moins un amide d'une alcanolamine et d'un acide gras en C₁₄-C₃₀ et un agent oxydant.

Par « composition prête à l'emploi », on entend, au sens de l'invention, la composition destinée à être appliquée telle quelle sur les fibres kératiniques, c'est-à-dire qu'elle peut être stockée telle quelle avant utilisation ou résulter du mélange extemporané de deux ou plusieurs compositions.

La demande décrit également un procédé de teinture d'oxydation des fibres kératiniques, et en particulier des fibres kératiniques humaines telles que les cheveux, consistant à appliquer sur les fibres une composition (A) contenant, dans un milieu approprié pour la teinture, au moins un colorant d'oxydation, au moins un alcool gras non oxyalkyléné, au moins un polymère associatif choisi parmi les polymères amphiphiles, et au moins un amide d'une alcanolamine et d'un acide gras en C₁₄-C₃₀, le rapport en poids de l'amide d'un alcanolamine et d'un acide gras en C₁₄-C₃₀ sur le polymère associatif étant compris entre 5 et 20, la couleur étant révélée à pH alcalin, neutre ou acide, à l'aide d'une composition (B) contenant au moins un agent oxydant, qui est mélangée juste au moment de l'emploi à la composition (A) ou qui est appliquée séquentiellement sans rinçage intermédiaire.

La demande décrit des dispositifs de teinture à plusieurs compartiments ou « kits » à plusieurs compartiments pour la teinture d'oxydation des fibres kératiniques, en particulier des fibres kératiniques humaines telles que les cheveux. Un dispositif peut comporter un premier compartiment contenant au moins un colorant d'oxydation, au moins un alcool gras non oxyalkyléné, au moins un polymère associatif choisi parmi les polymères amphiphiles, et au moins un amide d'une alcanolamine et d'un acide gras en C₁₄-C₃₀, le rapport en poids de l'amide d'un alcanolamine et d'un acide gras en C₁₄-C₃₀ sur le polymère associatif étant compris entre 5 et 20 et un deuxième compartiment contenant un agent oxydant.

D'autres caractéristiques, aspects, objets et avantages de l'invention apparaîtront encore plus clairement à la lecture de la description et des exemples qui suivent.

L'amide d'une alcanolamine et d'un acide gras en C₁₄-C₃₀ est de préférence choisi parmi les amides d'une alcanolamine en C₂-C₁₀ et d'un acide gras en C₁₄-C₃₀, et encore plus préférentiellement parmi les amides d'une alcanolamine en C₂-C₁₀ et d'un acide gras en C₁₄-C₂₂.

L'amide d'une alcanolamine et d'un acide gras en C₁₄-C₃₀ est de préférence choisi parmi :
- le diéthanolamide d'acide oléique, tel que l'amide commercialisé sous la dénomination commerciale MEXANYL® GT par la société CHIMEX,
- le monoéthanolamide d'acide myristique, tel que l'amide commercialisé sous la dénomination commerciale COMPERLAN® MM par la société COGNIS,
- le diéthanolamide d'acides gras de soja, tel que l'amide commercialisé sous la dénomination commerciale COMPERLAN® VOD par la société COGNIS,
- l'éthanolamide d'acide stéarique, tel que l'amide commercialisé sous la dénomination commerciale MONAMID® S par la société UNIQEMA,
- le monoisopropanolamide d'acide oléique, tel que l'amide commercialisé sous la dénomination commerciale WITCAMIDE® 61 par la société WITCO,
- le diéthanolamide d'acide linoléique, tel que l'amide commercialisé sous la dénomination commerciale PURTON® SFD par la société ZSCHIMMER SCHWARZ,
- le monoéthanolamide d'acide stéarique, tel que l'amide commercialisé sous la dénomination commerciale MONAMID® 972 par la société ICI/UNIQEMA,
- le monoéthanolamide d'acide béhénique, tel que l'amide commercialisée sous la dénomination commerciale INCROMIDE® BEM de CRODA,
- le monoisopropanolamide d'acide isostéarique, tel que l'amide commercialisé sous la dénomination commerciale WITCAMIDE® SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS,

L'amide d'une alcanolamine et d'un acide gras en C₁₄-C₃₀ est présent dans la composition dans des proportions en poids comprises de préférence entre 0,1 et 10%, et encore de préférence entre 1 et 5% du poids total de la composition.

L'alcool gras non oxyalkyléné selon l'invention peut être saturé ou insaturé, linéaire ou ramifié.

Parmi ces alcools gras on préfère ceux en C12-C22.

On peut citer parmi eux les alcools laurique, cétylique, stéarylique, oléïque, béhénique, linoléïque, undécylénique, palmitoléïque, arachidonique, érucique, et leurs mélanges.

L'alcool gras non oxyalkyléné est présent dans la composition dans des proportions en poids comprises de préférence entre 0,1 et 20%, et encore de préférence entre 1 et 10% du poids total de la composition.

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques, ainsi que leurs sels d'addition avec un acide.

On peut notamment citer :
- (I) les paraphénylènediamines de formule (I) suivante et leurs sels d'addition avec un acide : 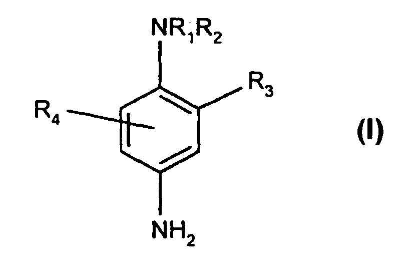
dans laquelle :
R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.
   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)-amino 2-chloro-aniline, la 2-β-hydroxyéthyl-paraphénylènediamine, la 2-fluoroparaphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
-(II) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 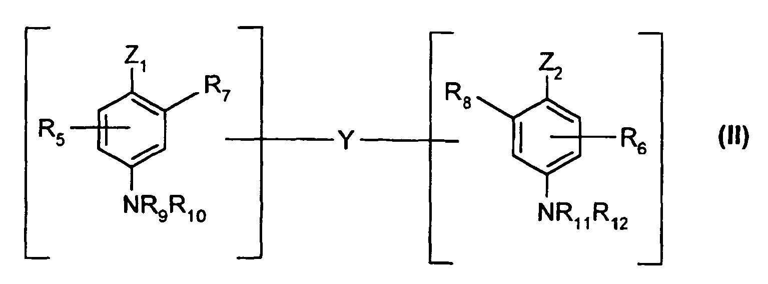 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.
   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (III) les para-aminophénols répondant à la formule (III) suivante, et leurs sels d'addition avec un acide : 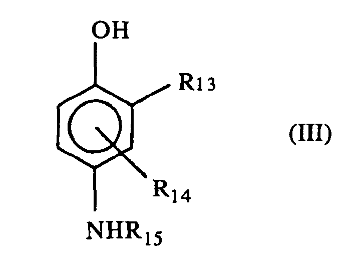 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).
   R₁₅ représente un atome d'hydrogène ou un radical alkyle en C₁-C₄.
   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, le N-méthyl paraminophénol et leurs sels d'addition avec un acide.
- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids du poids total de la composition et encore plus préférentiellement de 0,005 à 8% en poids de ce poids.

Les coupleurs utilisables dans le procédé de teinture selon l'invention sont ceux classiquement utilisés dans les compositions de teinture d'oxydation, c'est-à-dire les méta-aminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids du poids total de la composition, et encore plus préférentiellement de 0,005 à 5% en poids.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale de 0,001 à 20% et de préférence de 0,01 à 10% du poids total de la composition.

Les polymères associatifs sont des polymères dont les molécules sont capables, dans le milieu de formulation, de s'associer entre elles ou avec des molécules d'autres composés.

Les polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

Les polymères associatifs amphiphiles selon l'invention peuvent être choisis parmi les polymères associatifs non ioniques, anioniques, cationiques et amphotères.

Les polymères associatifs amphiphiles selon l'invention sont choisis parmi les polymères associatifs comportant au moins une chaîne grasse. La chaîne grasse comporte de préférence de 8 à 30 atomes de carbone, et encore plus préférentiellement de 10 à 30 atomes de carbone.

Parmi les polymères associatifs comportant au moins une chaîne grasse et de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = C R' CH₂ O Bₙ R (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
   Des polymères amphiphiles anioniques de ce type sont décrits et préparés, selon un procédé de polymérisation en émulsion, dans le brevet EP-0 216 479.
   Parmi ces polymères associatifs anioniques à chaîne grasse, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 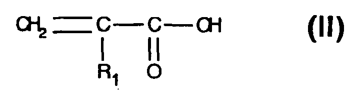 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante : 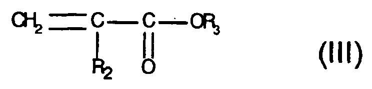 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques à chaîne grasse, on utilisera plus particulièrement des polymères formés à partir d'un mélange de monomères comprenant :
   (i) essentiellement de l'acide acrylique,
   (ii) un ester de formule (III) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ce type de polymères associatifs anioniques à chaîne grasse, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précedemment.
   Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX .
- (III) les terpolymères d'anhydride maléique/α-oléfine en C₃₀-C₃₈/maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1C4.

A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères associatifs à chaîne grasse de type non ionique, utilisés selon l'invention, sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
- (3) les copolymères de vinyl pyrrolidone et de monomères hydrophobes à chaîne grasse ;
   on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 8 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184, l'Aculyn 44 et l'Aculyn 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer. le produit ELFACOS T210 à chaîne alkyle en C₁₂₋₁₄ et le produit ELFACOS T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS. Les polyéthers polyuréthanes utilisables selon l'invention sont en particulier ceux décrits dans l'article de G. Fonnum, J. Bakke et Fk. Hansen - Colloid Polym. Sci 27.1, 380.389 (1993).

Les polymères associatifs à chaîne grasse de type cationique utilisés dans la présente invention sont choisis de préférence parmi les dérivés de cellulose quaternisée, les polyacrylates à groupements latéraux aminés non cycliques, les polyuréthanes cationiques, les polyvinyllactames cationiques et le terpolymère acrylique dont la constitution est donnée ci-après.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les hydroxyéthylcelluloses quaternisées modifiées par un groupement alkyle en C₁₂ ou C₁₈ tels que les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

Les polyacrylates à groupements latéraux aminés, quaternisés ou non, possèdent par exemple des groupements hydrophobes du type stéareth 20 (alcool stéarylique polyoxyéthyléné(20)).
Comme exemples de polyacrylates à chaînes latérales aminées, on peut citer les polymères 8781- 121B ou 9492-103 proposés par la société NATIONAL STARCH.

Les polyuréthanes associatifs cationiques selon la présente invention sont plus particulièrement choisis parmi les polyuréthanes amphiphiles associatifs cationiques, hydrosolubles ou hydrodispersibles.

Le terme "hydrosoluble" ou "soluble dans l'eau" concernant les polyuréthanes associatifs de la présente invention signifie que ces polymères ont une solubilité dans l'eau à température ambiante au moins égale à 1 % en poids, c'est-à-dire que jusqu'à cette concentration, aucun précipité ne peut être détecté à l'oeil nu et la solution est parfaitement limpide et homogène.

On entend par polyuréthanes "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

La famille de polyuréthanes amphiphiles cationiques selon l'invention a été décrite par la demanderesse dans la demande de brevet français N°-0009609; elle peut être représentée par la formule générale (Ia) suivante :

R-X-(P)ₙ-[L-(Y)ₘ]ᵣ-L'-(P')ₚ-X'-R' (Ia)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins un groupement hydrophobe.

Dans un mode de réalisation préféré des polyuréthanes de la présente invention, les seuls groupements hydrophobes sont les groupes R et R' aux extrémités de chaîne.

Une famille préférée de polyuréthanes amphiphiles cationiques est celle correspondant à la formule (Ia) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes amphiphiles cationiques est celle correspondant à la formule (Ia) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes amphiphiles cationiques est celle correspondant à la formule (Ia) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 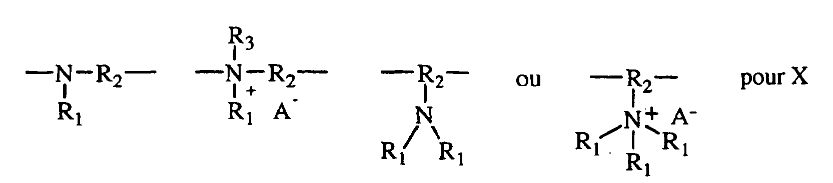 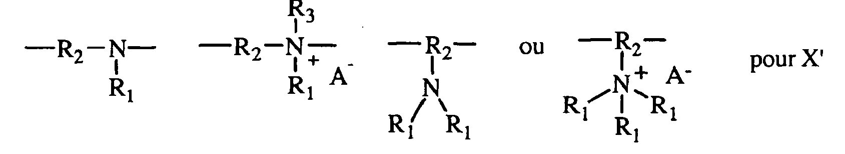 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 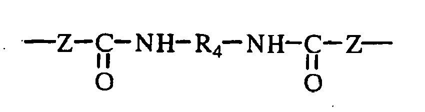 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 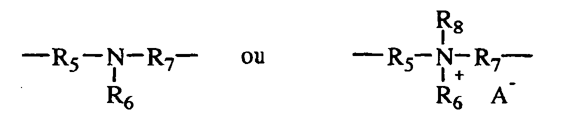 ou 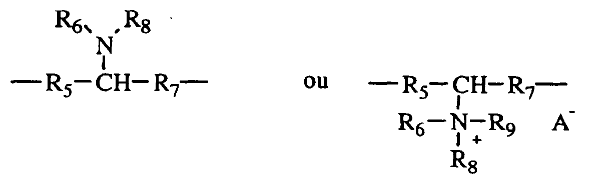 ou 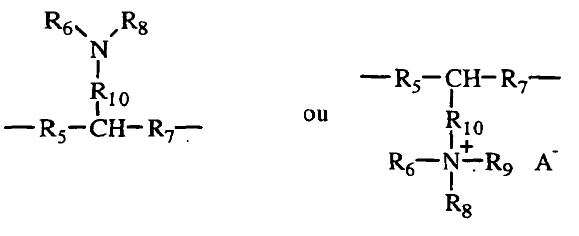 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (Ia) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (Ia) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine.
Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (Ia) est un diisocyanate correspondant à la formule :

O=C=N-R₄-N=C=O

dans laquelle R₄ est défini plus haut.
A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (Ia) est un composé hydrophobe destiné à former les groupes hydrophobes terminaux du polymère de formule (Ia).
Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné _-hydroxyle.
Le groupe hydrophobe du polyuréthane de formule (Ia) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (Ia) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.
Lesdits polyuréthanes associatifs cationiques sont hydrosolubles ou hydrodispersibles.

Les polymères poly(vinyllactame) cationiques selon l'invention comprennent :
- a) au moins un monomère de type vinyl lactame ou alkylvinyllactame;
- b) au moins un monomère de structures (Ib) ou (IIb) suivantes : 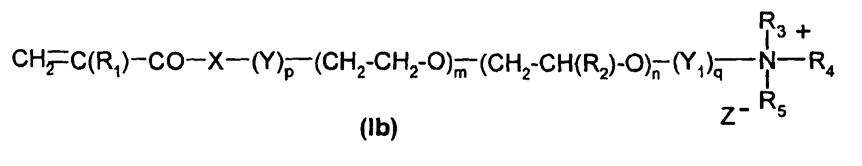 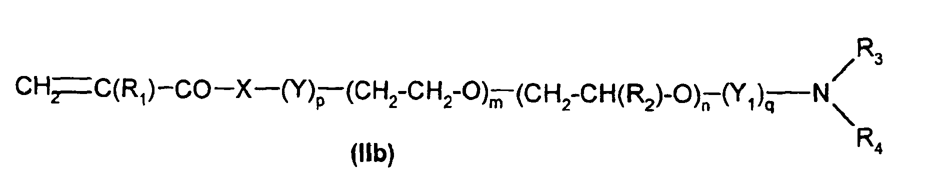 dans lesquelles :
   X désigne un atome d'oxygène ou un radical NR₆,
   R₁ et R₆ désignent, indépendamment l'un de l'autre, un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent, indépendamment l'un de l'autre, un atome d'hydrogène, un radical alkyle linéaire ou ramifié en C₁-C₃₀ ou un radical de formule (IIIb) : 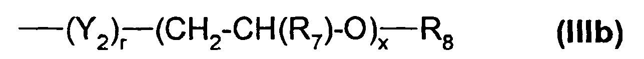
   Y, Y₁ et Y₂ désignent, indépendamment l'un de l'autre, un radical alkylène linéaire ou ramifié en C₂-C₁₆,
   R₇ désigne un atome d'hydrogène, ou un radical alkyle linéaire ou ramifié en C₁-C₄ ou un radical hydroxyalkyle linéaire ou ramifié en C₁-C₄,
   R₈ désigne un atome d'hydrogène ou un radical alkyle linéaire ou ramifié en C₁-C₃₀,
   p, q et r désignent, indépendamment l'un de l'autre, soit la valeur zéro, soit la valeur 1,
   m et n désignent, indépendamment l'un de l'autre, un nombre entier allant de 0 à 100,
   x désigne un nombre entier allant de 1 à 100,
   Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence le contre ion Z⁻. des monomères de formule (Ib) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.
Plus préférentiellement, le monomère b) est un monomère de formule (Ib) pour laquelle, encore plus préférentiellement, m et n sont égaux à zéro.

Le monomère vinyl lactame ou alkylvinyllactame est de préférence un composé de structure (IVb) : 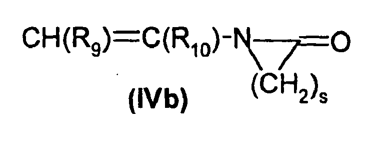 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (IVb) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (IVb),
b)-un monomère de formule (Ib) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (IIb) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b).
De tels polymères sont décrits dans la demande de brevet WO-00/68282.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone /diméthylaminopropylméthacrylamide/ tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.
La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.
Parmi les polymères amphiphiles cationiques selon l'invention, on peut aussi citer les terpolymères acryliques tels que décrits dans la demande de brevet EP- 1090 623 et qui sont constitués de :
- de 5 à 80% en poids, préférentiellement de 15 à 70% en poids et plus préférentiellement de 40 à 70% en poids, d'un monomère acrylate (a), choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b), choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di- (C₁-C₄)alkylamino(C₁-C₄)alkyl (méth)acrylamide;
- de 0,1 à 30% en poids, préférentiellement de 0,1 à 10% en poids d'un monomère (c), choisi parmi (i)un uréthane produit par réaction entre un isocyanate insaturé monoéthylénique et un tensioactif non ionique à terminaison C₁-C₄ alkoxy; (ii) un copolymère à blocs de 1,2-butylène oxyde et de 1,2-éthylène oxyde; (iii) un monomère tensioactif insaturé éthylénique copolymérisable obtenu par condensation d'un tensioactif non-ionique avec un acide carboxylique insaturé α,β-éthylénique ou son anhydride; (iv) un monomère tensioactif
choisi parmi les produits de réaction de type urée d'un monoisocyanate insaturé monoéthylénique avec un tensioactif non-ionique présentant une fonction amine; (v)un éther de (méth)allyle de formule CH₂= CR₁CH₂OAₘBₙAₚR₂ dans lequel R₁ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur à 100, m et p désignent zéro ou un nombre entier inférieur à n et R₂ est un groupe hydrophobe d'au moins 8 atomes de carbone et préférentiellement en C₈-C₃₀; et (vi)un monomère non-ionique de type uréthane produit par réaction d'un tensioactif non ionique monohydrique avec un isocyanate insaturé monoéthylénique;
les pourcentages en poids de monomères étant basés sur le poids total des monomères constituant le terpolymère.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropyl-acrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou dicarboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.
Les terpolymères acryliques peuvent ainsi être choisis parmi les terpolymères acryliques constitués d'acrylates, d'amino(méth)acrylates et d'itaconate d'alkyle en C₁₀-C₃₀ polyoxyéthyléné à 20 moles d'oxyde d'éthylène.
Des méthodes conventionnelles pour préparer ces terpolymères acryliques sont connues de l'homme du. métier. De telles méthodes incluent la polymérisation en solution, la polymérisation par précipitation et la polymérisation en émulsion. Des terpolymères conformes à l'invention et leurs méthodes de préparation sont notamment décrits dans les demandes EP-A-0824914 et EP-A-0825200.
Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de Matière Active.
En plus de ces monomères, les terpolymères peuvent contenir d'autres monomères qui permettent de réticuler lesdits terpolymères. Ces monomères sont utilisés dans des proportions assez faibles, jusqu'à 2% en poids par rapport au poids total des monomères utilisés pour préparer les terpolymères. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes.
Des monomères de réticulation peuvent notamment être des divinyl-benzènes, des trivinyl-benzènes, le 1,2,4-trivinylcyclohexène, le 1,5-hexadiène, le 1,5,9-décatriène, le 1,9-décadiène, le 1,5-heptadiène, des diallyl phthalates, de l'éthylène glycol diméthacrylate, des polyéthylène glycol diméthacrylates, des penta- et tétra-acrylates, des triallyl pentaérythritols, des octa-allyl saccharoses, des cycloparaffines, des cyclooléfines et du N-méthylène-bis-acrylamide.

Les polymères associatifs selon l'invention peuvent également être choisis parmi les polymères associatifs amphotères.
On désigne généralement par "polymères amphotères", des polymères qui comportent des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères utilisés selon l'invention comportent en outre au moins une chaîne grasse ayant de 8 à 30 atomes de carbone, et peuvent être choisis par exemple parmi les polymères dérivés d'acide polyaspartique comportant au moins une chaîne grasse ayant de 8 à 30 atomes de carbone, tels que ceux :
- décrits et préparés dans la demande de brevet EP- 0767 191. De tels polymères sont préparés de façon connue, par réaction en milieu solvant, de polysuccinimide (PSI) sur des amines à chaîne grasse (en C₈-C₂₄) , en la présence ou en l'absence de catalyseur basique tel que par exemple d'amines tertiaires aliphatiques, puis amphotérisation du produit obtenu par réaction avec un acide organique halogéné.
   Parmi les amines à chaîne grasse en C₈-C₂₄ que l'on fait réagir avec la PSI, on peut citer notamment l'octylamine, la nonylamine, la décylamine, la dodécylamine, la tétradécylamine, l'hexadécylamine, l'octadécylamine, l'octadécénylamine, l'eicosyldécylamine, l'octynylamine, la décénylamine, la dodécénylamine, la tétradécénylamine, l'hexadécénylamine, l'octadécénylamine, et l'eicosénylamine.
   Des exemples de tels polymères sont préparés par réaction de la PSI sur de la n-laurylamine ou de la n-stéarylamine en présence de N,N-diméthyl-1,3-propane diamine comme catalyseur basique, suivie d'une amphotérisation du produit obtenu par réaction avec le monochloroacétate de potassium. Ces polymères sont préparés avec de plus amples détails en pages 13 à 20 (lignes 1-4) et dans les exemples 1 à 5 en pages 28 à 34 (lignes 1-4) de la dite demande de brevet EP- 0767 191.
- décrits et préparés dans la demande de brevet EP- 0 884 344. De tels polymères sont préparés par réaction en milieu solvant, d'ammoniac gazeux sur un monomaléate d'alkyl ou alcényl en C₈-C₂₄ , sous pression réduite et à la température de 120-140°C pendant 4 à 6 heures.
   Les radicaux alkyl ou alcényl en C₈-C₂₄ peuvent notamment être choisis parmi les radicaux suivants linéaires ou ramifiés : décyl, dodécyl, tétradécyl, hexadécyl, octadécyl, oléyl.
   Des exemples de tels polymères comprennent les polymères à unités acide aspartique et à unités aspartate de décyl, les polymères à unités acide aspartique et à unités aspartate de dodécyl, les polymères à unités acide aspartique et à unités aspartate de cétyl, les polymères à unités acide aspartique et à unités aspartate de stéaryl, les polymères à unités acide aspartique et à unités n-décylaspartamide, décrits dans les exemples 1 à 6 de ladite demande de brevet.
- décrits et préparés dans la demande de brevet EP- 0 959 094. De tels polymères sont préparés par réaction en milieu solvant, d'ammoniac gazeux sur un monoamide d'acide maléique, polyoxyalkyléné et modifié hydrophobe par une chaîne alkyle ou alcényle en C₈-C₃₀ linéaire ou ramifiée, éventuellement en mélange avec un monoester d'acide maléique.
   Un exemple de polymère ainsi préparé est décrit dans l'exemple 2 page 11 de ladite demande de brevet.
- décrits et préparés dans la demande de brevet EP- 0 959 090. De tels polymères modifiés hydrophobes et de poids moléculaire élevé sont obtenus à partir de dérivés d'acide maléique et d'ammoniac gazeux et d'alcools ou d'amines di- ou poly-fonctionnels.
   Des exemples de copolymères à unités acide aspartique et aspartate de cétyle ou à unités acide aspartique et aspartate de cétyle sont respectivement donnés dans les exemples 3 et 5 de ladite demande de brevet.
- ou encore ceux décrits et préparés dans la demande de brevet EP- 0 959 091. De tels polymères modifiés hydrophobes sont préparés à partir de monoester ou monoamide d'acide maléique et d'ammoniac gazeux.
   Des exemples de tels copolymères sont donnés dans les exemples 1, 2, 3, et 5 de ladite demande de brevet.

De préférence selon la présente invention, les polymères amphotères comportant au moins une chaîne grasse ayant de 8 à 30 atomes de carbone sont choisis parmi ceux comportant au moins un motif cationique non cyclique. Et plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.
Lesdits polymères amphotères à chaîne grasse préférés selon l'invention comprennent, ou sont préparés en copolymérisant :
1) au moins un monomère de formule (Ia) ou (Ib): 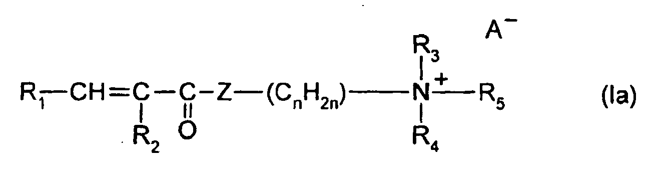 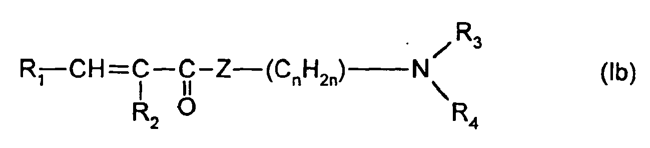 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure;
2) au moins un monomère de formule (II)

   R₆-CH=CR₇-COOH (II)

   dans laquelle, R₆ et R₇, identiques ou différents, représentent un atome d'hydrogène ou un radical méthyle;
   et
3) au moins un monomère de formule (III) :

   R₆ - CH=CR₇-COXR₈ (III)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (Ia), (Ib) ou (III) comportant au moins une chaîne grasse.
Les monomères de formule (Ia) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères amphotères à chaîne grasse selon l'invention comprennent de préférence de 1 à 10 moles% du monomère comportant une chaîne grasse (monomère de formule (Ia), (Ib) ou (III)), et de préférence de 1,5 à 6 moles % .

Les poids moléculaires moyens en poids des polymères amphotères à chaîne grasse selon l'invention peuvent varier de 500 à 50.000.000 et sont de préférence compris entre 10.000 et 5 000 000.

Les polymères amphotères à chaîne grasse selon l'invention peuvent également contenir d'autre monomères tels que des monomères non ioniques et en particulier tels que les acrylates ou méthacrylates d'alkyle en C₁-C₄.

Des polymères amphotères à chaîne grasse selon l'invention sont par exemple décrits et préparés dans la demande de brevet WO9844012.

Parmi les polymères amphotères à chaîne grasse selon l'invention, on préfère les terpolymères acide acrylique/chlorure de (méth)acrylamidopropyl triméthyl ammonium/ méthacrylate de stéaryle.

Dans la composition de teinture d'oxydation selon l'invention, on préfère utiliser un polymère associatif amphiphile à chaîne grasse de type cationique ou non ionique, et encore plus préférentiellement de type cationique. Encore plus préférentiellement, le polymère associatif amphiphile est choisi parmi les polyuréthanes cationiques.

Le ou les polymères associatifs amphiphiles sont présents dans la composition à des teneurs en poids comprises de préférence entre 0,05 et 10%, et encore de préférence entre 0,1 et 5% du poids total de la composition.
Le rapport en poids de l'amide d'une alcanolamine et d'un acide gras en C₁₄-C₃₀ sur le polymère associatif est compris entre 5 et 20.

Le rapport en poids de l'amide d'une alcanolamine et d'un acide gras en C₁₄-C₃₀ sur l'alcool gras non oxyéthyléné est de préférence compris entre 0,1 et 10, et encore de préférence entre 0,5 et 5.

La composition (A) et/ou la composition (B) peuvent en outre plus particulièrement contenir, au moins un polymère substantif amphotère ou cationique différent des polymères associatifs de l'invention.

Au sens de la présente invention, l'expression "polymère cationique" désigne tout polymère contenant des groupements cationiques et/ou des groupements ionisables en groupements cationiques.

Les polymères substantifs cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer
(1) Les homopolymères ou copolymères dérivés d'esters ou d'amides acryliques ou méthacryliques et comportant au moins un des motifs de formules (I), (II), (III) ou (IV) suivantes: 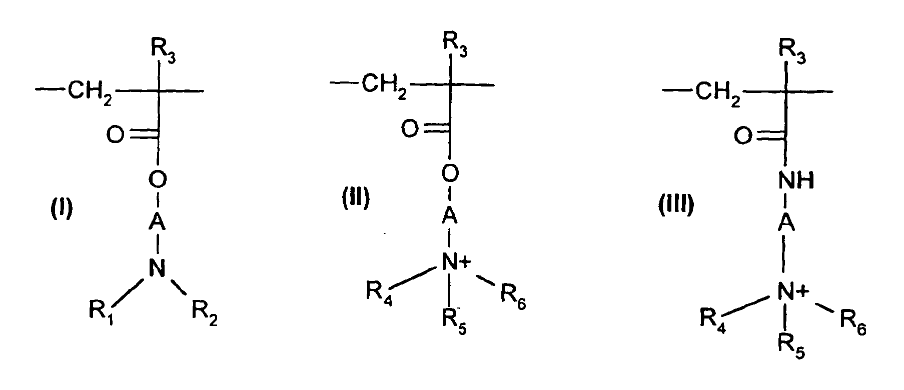 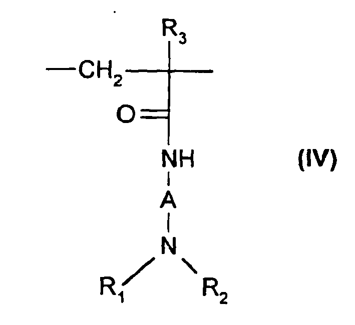 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 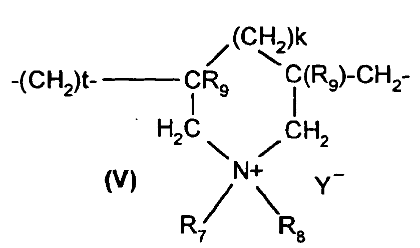 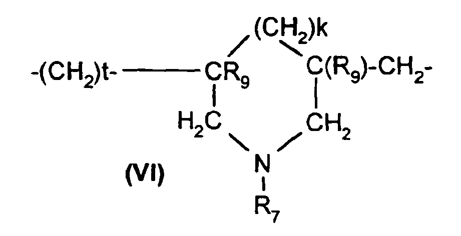 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 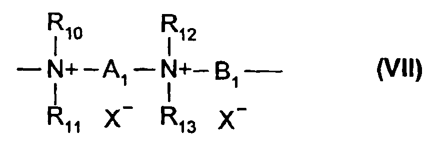 formule (VII) dans laquelle :
   R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 6 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule :

         -NH-CO-NH- .
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 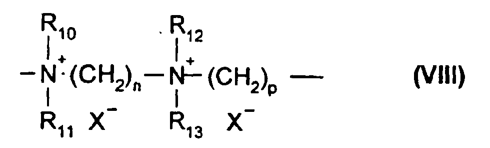 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs de formule (IX) : 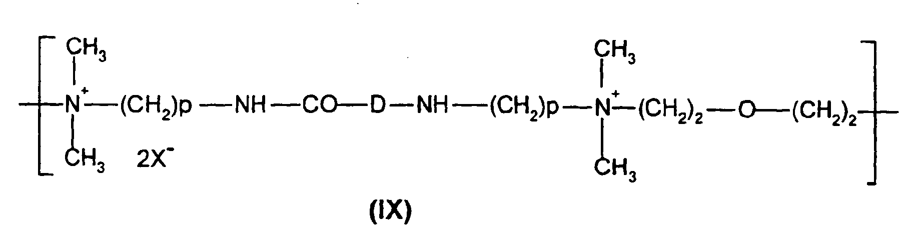 dans laquelle :
   p désigne un nombre entier variant de 1 à 6 environ,
   D peut être nul ou peut représenter un groupement ―(CH₂)ᵣ ―CO ― dans lequel r désigne un nombre égal à 4 ou à 7, et
   X⁻ est un anion dérivé d'un acide minéral ou organique.
   Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.
   Parmi ces polymères, on préfère ceux de masse moléculaire mesurée par RMN du Carbone 13 inférieure à 100000, et dans la formule de laquelle :
   p est égal à 3, et,
   a) D représente un groupement ―(CH₂)₄ ―CO ― , X désigne un atome de chlore, la masse moléculaire mesurée par RMN du Carbone 13 ( RMN¹³C ) étant d'environ 5600 ; un polymère de ce type est proposé par la société MIRANOL sous le nom de MIRAPOL-AD1,
   b) D représente un groupement ―(CH₂)₇ ―CO ― , X désigne un atome de chlore, la masse moléculaire mesurée par RMN du Carbone 13 ( RMN¹³C ) étant d'environ 8100 ; un polymère de ce type est proposé par la société MIRANOL sous le nom de MIRAPOL-AZ1,
   c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
   d) un " Block Copolymer " formé de motifs correspondant aux polymères décrits aux alinéas a) et c), proposé par la société MIRANOL sous les noms MIRAPOL-9,
   (masse moléculaire RMN¹³C, environ 7800) MIRAPOL-175, (masse moléculaire RMN¹³C, environ 8000) MIRAPOL-95, (masse moléculaire RMN¹³C, environ 12500).
   Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13,
   ( RMN¹³C ) étant d'environ 25500.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 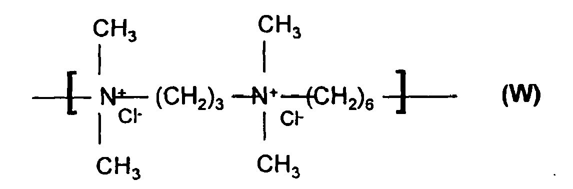 et notamment ceux dont le poids moléculaire, déterminé par chromatographie par perméation de gel, est compris entre 9500 et 9900; 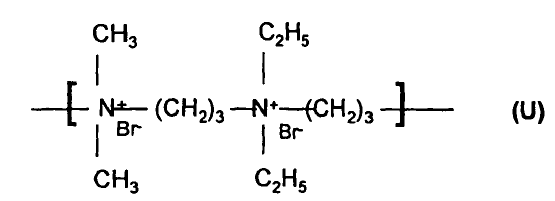 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères substantifs amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 6 atomes de carbone et plus particulièrement le N-éthylacrylamide, le N-tertiobutyl-acrylamide, ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone
   des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 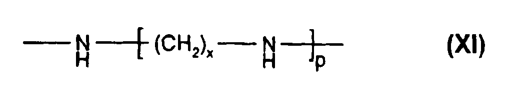 où x=2 et p=2 ou 3, ou bien x=3 et p=2
      ce radical dérivant de la diéthylène triamine, de la triéthylène tétraamine
      ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 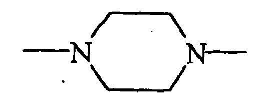
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 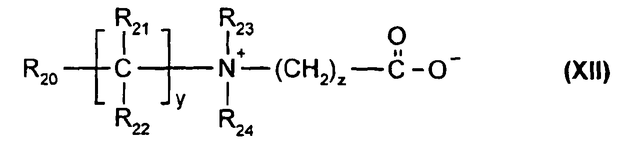 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 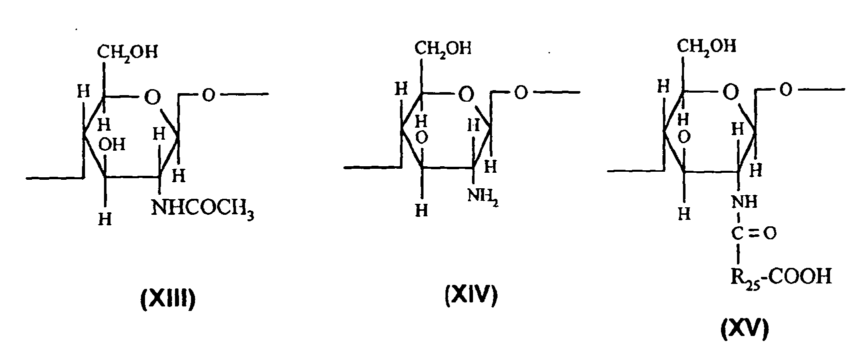 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 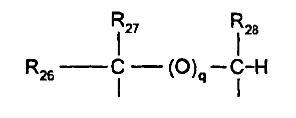 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 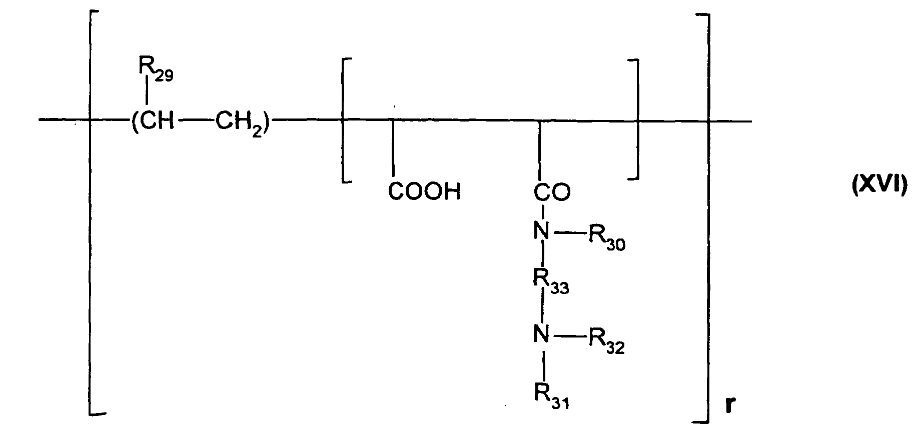 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule :- R₃₃-N(R₃₁)₂, R₃₃ représentant un groupement -CH₂-CH₂- , - CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 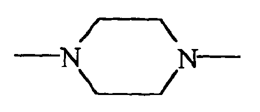 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)

      où D désigne un radical 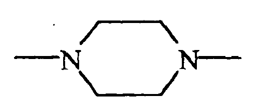 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.
   Les polymères amphotères particulièrement préférés selon l'invention sont ceux de la famille (1).
   Selon l'invention, le ou les polymères substantifs amphotères peuvent représenter de 0,01 % à 10 % en poids, de préférence de 0,05 % à 5 % en poids, et encore plus préférentiellement de 0,1 % à 3 % en poids, du poids total de la composition.
   Les compositions de l'invention comprennent de préférence un ou plusieurs tensioactifs.
   Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle linéaire ou ramifié en C₅-C₂₀ provenant par exemple d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle, linéaire ou ramifié, saturé ou non, en C₅-C₂₀ d'un acide R₉ -COOH présent par exemple dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Les compositions selon l'invention peuvent également contenir des agents d'ajustement de la rhéologie non associatifs tels que les épaississants cellulosiques
(hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.

Ces épaississants d'appoint peuvent représenter de 0,01 à 10% en poids du poids total de la composition.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol.

Les solvants peuvent alors être présents dans des concentrations comprises entre environ 0,5 et 20% et, de préférence, entre environ 2 et 10% en poids par rapport au poids total de la composition.

La composition (A) peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol.

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant de 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition (B), l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, de 1 à 40 volumes, et encore plus préférentiellement de 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale (A) et de la composition oxydante (B), est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 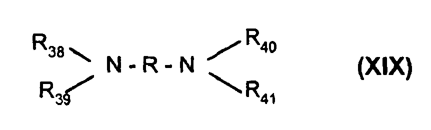 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture consiste, de préférence, à appliquer la composition prête à l'emploi, réalisée extemporanément au moment de l'emploi à partir des compositions (A) et (B) décrites ci-avant, sur les fibres kératiniques sèches ou humides, et à la laisser agir pendant un temps de pause variant, de préférence, de 1 à 60 minutes, et plus préférentiellement de 10 à 45 minutes, à rincer les fibres, puis éventuellement à les laver au shampooing, puis à les rincer à nouveau, et à les sécher.

Une variante de ce procédé consiste à appliquer séquentiellement de manière décalée ou simultanée sur les fibres kératiniques sèches ou humides avec un éventuel rinçage intermédiaire une composition décrite ci-dessus et une composition comprenant un agent oxydant et à laisser agir lesdites compositions pendant un temps de pose variant de 1 à 60 minutes puis à rincer les fibres, puis éventuellement à les laver au shampooing puis à les rincer à nouveau et à les sécher.

L'exemple suivant est destiné à illustrer l'invention.

On a préparé la composition suivante (quantités exprimées en pourcentages en poids)

| | |
|---|---|
| Mélange d'alcools linéaires en C18 à C24 (C18/C20/C22/C24 : 7/57/30/6- teneur en alcool > 95%) | 3 |
| Alcool stéarylique oxyéthyléné (2OE) | 4,5 |
| Alcool stéarylique oxyéthyléné (21 OE) | 1,75 |
| Acide oléique | 2,6 |
| Polyuréthane cationique à chaîne grasse obtenu à partir de la condensation de 1,3bis(isocyanatométhylcyclohexane), de N,N-diméthyléthanolamine quaternisée avec le bromododécane, de N,Ndiméthyléthanolamine et de Polyoxyéthylène de poids moléculaire 10000 | 0,2 |
| Acide polyacrylique réticulé | 0,4 |
| Hydroxypropyl méthyl cellulose | 0,2 |
| Monoéthanolamide d'acide stéarique | 3 |
| Merquat 100 en solution aqueuse à 40% | 4 |
| Polymère cationique de formule (W) | 2 |
| Propylène glycol | 2 |
| Métabisulfite de sodium | 0,71 |
| EDTA (acide éthylènediamine tétra-acétique) | 0,2 |
| Ter-butyl hydroquinone | 0,3 |
| 1,4-diaminobenzène | 0,2 |
| Para-aminophénol | 1,2 |
| 1,3-dihydroxybenzène | 0,1 |
| 1-hydroxy-3-amino-benzène | 0,2 |
| 1-méthyl-2-hydroxy-4-β-hydroxyéthylamino-benzène | 0,8 |
| Monoéthanolamine | 1 |
| Ammoniaque à 20% de NH₃ | 11 |
| Parfum q.s | |
| Eau déminéralisée q.s.p | 100 |

Cette composition est mélangée au moment de l'emploi à une composition oxydante sous forme d'émulsion contenant comme agent oxydant 7,5% de peroxyde d'hydrogène à raison de 1 partie en poids de composition colorante pour 1,5 parties en poids de composition oxydante. On applique le mélange obtenu sur des mèches de cheveux naturels à 90% de blancs et on laisse poser 30 minutes. Après rinçage, lavage au shampooing et séchage, on obtient des cheveux teints dans une nuance soutenue châtain clair rouge cuivré.

## Revendications

1. Composition pour la teinture d'oxydation des fibres kératiniques, en particulier des fibres kératiniques humaines, telles que les cheveux, **caractérisée en ce qu'**elle comprend, dans un milieu approprié pour la teinture,
a) au moins un colorant d'oxydation,
b) au moins un alcool gras non oxyalkyléné,
c) au moins un polymère associatif choisi parmi les polymères amphiphiles, et
d) au moins un amide d'une alcanolamine et d'un acide gras en C₁₄-C₃₀; le rapport en poids de l'amide d'un alcanolamine et d'un acide gras en C₁₄-C₃₀ sur le polymère associatif étant compris entre 5 et 20.

2. Composition selon la revendication 1, **caractérisée en ce que** l'amide d'une alcanolamine et d'un acide gras en C₁₄-C₃₀ est choisi parmi les amides d'une alcanolamine en C₂-C₁₀ et d'un acide gras en C₁₄-C₃₀, et de préférence parmi les amides d'une alcanolamine en C₂-C₁₀ et d'un acide gras en C₁₄-C_{22.}

3. Composition selon la revendications 1 ou 2, **caractérisée en ce que** l'amide d'une alcanolamine et d'un acide gras en C₁₄-C₃₀ est choisi parmi
- le diéthanolamide d'acide oléique,
- le monoéthanolamide d'acide myristique,
- le diéthanolamide d'acides gras de soja,
- l'éthanolamide d'acide stéarique,
- le monoisopropanolamide d'acide oléique,
- le diéthanolamide d'acide oléique,
- le monoéthanolamide d'acide stéarique,
- le monoéthanolamide d'acide béhénique,
- le monoisopropanolamide d'acide isostéarique,
- le diéthanolamide d'acide érucique,
- le monoéthanolamide d'acide ricinoléique.

4. . Composition selon la revendication 3, **caracterisée en ce que** l'amide d'une alcanolamine et d'un acide gras en C₁₄-C₃₀ est choisi parmi :
- le diéthanolamide d'acide oléique
- le monoéthanolamide d'acide myristigue,
- le diéthanolamide d'acide gras de soja,
- l'éthanolamide d'acide stéarique,
- le diéthanolamide d'acide oléique,
- le monoéthanolamide d'acide stéarique,
- le monoéthanolamide d'acide béhénique,
- le diéthanolamide d'acide érucique,
- le monoéthanolamide d'acide ricinoléique.

5. Composition selon la revendication 1 ou 2, **caractérisé en ce que** l'amide d'une alcanolamine et d'un acide gras en C₁₄-C₃₀ est présent dans la composition dans des proportions en poids comprises entre 0,1 et 10%, et de préférence entre 1 et 5% du poids total de la composition.

6. Composition selon l'une quelconque des revendications précédentes, **caractérisée en ce que** l'alcool gras non oxyalkyléné est choisi parmi les alcools laurique, cétylique, stéarylique, oléique, béhénique, linoléïque, undécylénique, palmiltoléïque, arachidonique, érucique, et leurs mélanges.

7. Composition selon l'une quelconque des revendications précédentes, **caractérisée en ce que** l'alcool gras non oxyalkyléné est présent dans la composition dans des proportions en poids comprises entre 0,1 et 20%, et de préférence entre 1 et 10% du poids total de la composition.

8. Composition selon l'une quelconque des revendications précédentes, **caractérisée en ce que** le colorant d'oxydation est choisi parmi les bases d'oxydation et/ou les coupleurs.

9. Composition selon la revendication 8, **caractérisée en ce qu'**elle comprend au moins une base d'oxydation.

10. Composition selon la revendication 8 ou 9, **caractérisée en ce que** les bases d'oxydation sont choisies parmi les ortho- et para-phénylènediamines, les bases doubles, les ortho- et para-aminophénols, les bases hétérocycliques, ainsi que les sels d'addition de ces composés avec un aride.

11. Composition selon la revendication 10, **caractérisée en ce que** les para-phénylènediamines sont choisies parmi les composés de formule (I) suivante : 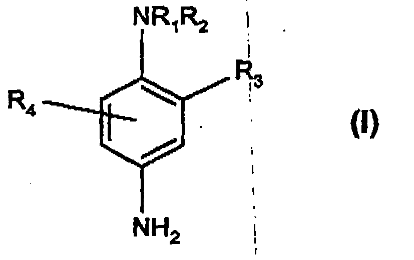 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle on 4'-aminophényle ;
- R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido :
- R₃ représente un atome d'hydrogène un atome d'halogène, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

12. Composition selon la revendication 10, **caractérisée en ce que** les bases doubles sont choisies parmi les composés de formule (II) suivante : 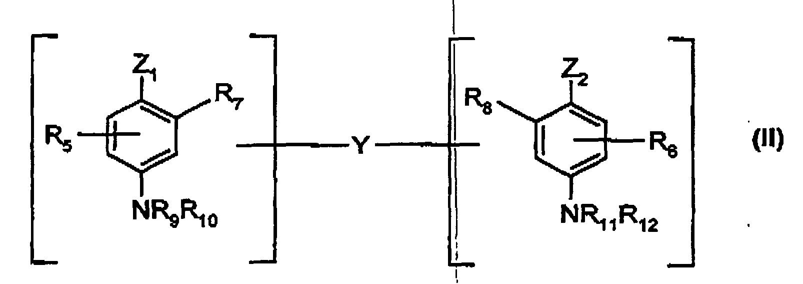 dans laquelle:
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un plusieurs groupements azotés et/ou par un ou plusieurs hétéroatomes, et éventuellement substituée par un plusieurs radicaux hydroxyle ou alcoxy en C₁-C₆ ;
- R₅ et R₆ représentent un atome d'hydrogène ou d'halogène, un radical alkyle en C₁-G₄, monohydroxyalkyle en C₁-C₄, polyhydroxyalkyle en C₂-C₄, aminoalkyle en C₁-C₄ ou un bras de liaison Y ;
- R₇, R₈, R₉, P₁₀, R₁₁ et R₁₂, identiques ou différents, représentent un atome d'hydrogène, un bras de liaison Y ou un radical alkyle en C₁-C₄;
étant entendu que les composés de formule (II) ne comportent qu'un seul bras de liaison Y par molécule.

13. Composition selon la revendication 10, **caractérisée en ce que** les para-aminophénols sont choisis parmi les composés de formule (III) suivante ; 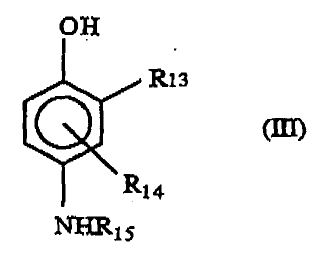 dans laquelle :
R₁₃ représente un atome d'hydrogène, un atome d'halogène, un radical alkyle en C₁-C₄. monohydroxyalkyle en C₁-C₄, alcoxy(G₁-C₄)alkyle(C₁-C₄) ou aminoalkyle en C₁-C₄, ou hydroxyalkyl (C₁-C₄)aminoalkyle en C₁-C₄,
R₁₄ représente un atome d'hydrogène, un atome d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
R₁₅ représente un atome d'hydrogène ou un radical alkyle en C₁-C₄.

14. Composition selon la revendication 10, **caractérisée en ce que** les bases hétérocycliques sont choisies parmi les dérivés pyridiniques, les dérivés pyrimidiniques, les dérivés pyrazoliques.

15. Composition selon l'une quelconque des revendications 8 à 14, **caractérisée en ce que** les bases d'oxydation représentent de 0,0005 à 12%, et de préférence de 0,005 à 8% en poids du poids total de la composition.

16. Composition selon la revendication 8, **caractérisée en ce que** les coupleurs sont choisis parmi les métaphénylènediamines, les métaaminophénols, les métadiphénols, les coupleurs hérérocycliques, et les sels d'addition de ces composés avec un acide.

17. Composition selon la revendication 8 ou 16, **caractérisée en ce que** les coupleurs représentent de 0,0001 à 10%, et de préférence de 0,005 à 5% en poids du poids total de la composition.

18. Composition selon l'une quelconque des revendications 8 à 17, **caractérisée en ce** les sels d'addition avec un acide des colorants d'oxydation sont choisis parmi les chlorhydrates, les bromhydrates, les sulfates, les tartrates, les lactates et les acétates.

19. Composition selon l'une quelconque des revendications précédentes, **caractérisée en ce qu'**elle comprend en outre des colorants directs.

20. Composition selon l'une quelconque des revendications précédentes, **caractérisée en ce que** le polymère amphiphile associatif est choisi parmi les polymères associatifs non ioniques, anioniques, cationiques et amphotères.

21. Composition selon la revendication 20, **caractérisée en ce que** le polymère associatif est un polymère associatif anionique à chaîne grasse comportant au moins un motif hydrophile et au moins un motif éther d'allyle à chaîne grasse.

22. Composition selon la revendication 21, **caractérisée en ce que** le motif hydrophile est un monomère anionique insaturé éthylénique et de préférence un acide carboxylique vinylique.

23. Composition selon la revendication 21, **caractérisée en ce que** le motif éther d'allyle à chaîne grasse est un monomère de formule (I) suivante
CH₂ = C R' CH₂ O Bₐ R (I)
dans laquelle R' désigne H ou CH3, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone.

24. Composition selon la revendication 21, **caractérisée en ce que** le polymère associatif anionique à chaîne grasse comporte au moins un motif hydrophile de type acide carboxylique insaturé oléfinique et au moins un motif hydrophobe de type ester d'alkyl(C₁₀-C₃₀) d'acide carboxylique insaturé.

25. Composition selon la revendication 24, **caractérisée en ce que** le motif hydrophile de type acide carboxylique insaturé oléfinique correspond au monomère de formule (II) suivante : 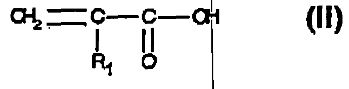 dans laquelle R1 désigne H ou CH₃ on C₂H₅, et dont le motif hydrophobe de type ester d'alkyl(C₁₀-C₃₀) d'acide carboxylique insaturé correspond au monomère de formule (III) suivante : 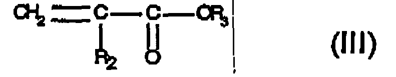 dans laquelle R₂ désigne H ou CH₃ ou C₂H₅, R₃ désignant un radical alkyle en C₁₀-C₃₀, et de préférence en C₁₂-C₂₂.

26. Composition selon la revendication 21, **caractérisé en ce que** le polymère associatif à chaîne grasse est un terpolymère d'anhydride maléique/α-oléfine en C₃₀-C₃₈/maléate d'alkyle.

27. Composition selon la revendication 21, **caractérisée en ce que** le polymère associatif à chaîne grasse est un terpolymère acrylique comprenant :
(a) environ 20% à 70% en poids d'un acide carboxylique à insaturation α,β-monoéthylénique,
(b) environ 20% à 80% en poids d'un monomère à insaturation α,β-monoéthylénique non tensio-actif différent de (a),
(c) environ 0,5% à 60% en poids d'un mono-uréthane non-ionique qui est le produit de la réaction d'un tensio-actif monohydrique avec un monoisocyanate à insaturation monoéthylénique.

28. Composition selon la revendication 21, **caractérisée en ce que** le polymère associatif à chaîne grasse est choisi parmi les copolymères comportant parmi leurs monomères un acide carboxylique à insaturation α,β-monoéthylénique et un ester d'acide carboxylique à insaturation α,β-monoéthylénique et d'un alcool gras oxyalkyléné.

29. Composition selon la revendication 20, **caractérisée en ce que** le polymère associatif à chaîne grasse non ionique est choisi dans le groupe formé par :
(1) les celluloses modifiées par des groupements comportant au moins une chaîne grasse ;
(2) les hydroxypropylguars modifiés par des groupements comportant au moins une chaîne grasse ;
(3) les polyuréthanes polyéthers comportant dans leur chaîne, à la fois des séquences hydrophiles de nature polyoxyéthylénée et des séquences hydrophobes qui sont des enchaînements aliphatiques seuls et/ou des enchaînements cycloaliphatiques et/ou aromatiques ;
(4) les copolymères de vinyl pyrrolidone et de monomères hydrophobes à chaîne grasse ;
(5) les copolymères de méthacrylates ou d'acrylates d'alkyles en C₁-C₆ et de monomères amphiphiles comportant au moins une chaîne grasse ;
(6) les copolymères de méthacrylates ou d'acrylates hydrophiles et de monomères hydrophobes comportant au moins une chaîne grasse ;
(7) les polymères à squelette aminoplaste éther possédant au moins une chaîne grasse.

30. Composition selon la revendications 29, **caractérisée en ce que** les polyéthers polyuréthanes comportent au moins deux chaînes lipophiles hydrocarbonées, ayant de 8 à 30 atomes de carbone, séparées par une séquence hydrophile, les chaînes hydrocarbonées étant des chaînes pendantes ou des chaînes en bout de séquence hydrophile.

31. Composition selon la revendication 29, **caractérisée en ce que** les polyéthers polyuréthanes sont multiséquencés, et de préférence sous forme de tribloc.

32. Composition selon la revendication 20, **caractérisée en ce que** le polymère associatif est un polymère cationiques comportant au moins une chaîne grasse et choisi parmi :
(i) les celluloses quaternisées modifiées par des groupements comportant au moins une chaîne grasse,
(ii) les hydroxyéthylcelluloses quaternisées modifiées par des groupements comportant au moins une chaîne grasse,
(iii) les polyuréthannes cationiques,
(iv) les polyvinyllactames cationiques,
(v) le terpolymère acrylique constitué d'acrylates, d'amino(méth)acrylates et d'itaconate d'alkyle en C₁₀-C₃₀ polyoxyéthyléné à 20 moles d'oxyde d'éthylène.

33. Composition selon la revendication 32, **caractérisée en ce que** les groupes alkyle des cellulose ou des hydroxyéthylcelluloses quaternisées comportent de 8 à 30 atomes de carbone.

34. Composition selon la revendication 32 ou 33, **caractérisée en ce que** le polymère amphiphile cationique est une hydroxyéthylcellulose quaternisée modifiée par un groupement alkyle en C₁₂ ou C₁₈.

35. Composition selon la revendication 32, **caractérisée en ce que** le polyuréthane amphiphile cationique est un polymère de formule (Ia) suivante :
R-X-(P)ₙ-[L-(Y)ₘ]ᵣ-L'-(P')ₚ-X'-R' (Ia)
dans laquelle : ,
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophxle ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternitsée et au moins un groupement hydrophobe.

36. Composition selon la revendication 28, **caractérisée en ce que** le polymère amphotère comporte au moins une chaîne grasse ayant de 8 à 30 atomes de carbone et au moins un motif cationique non cyclique.

37. Composition selon la revendication 36, **caractérisée en ce que** le polymère amphotère comprend de 1 à 20 moles% de monomère comportant une chaîne grasse, par rapport au nombre total de moles de monomères.

38. Composition selon la revendication 36 ou 37, **caractérisée en ce que** les polymères amphotères comprennent :
1) au moins un monomère de formule (Ia) ou (Ib): 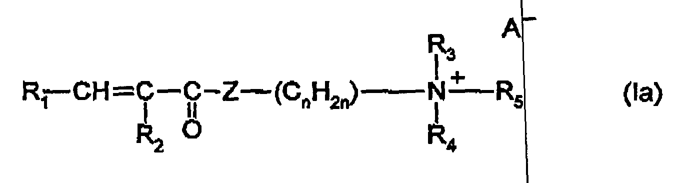 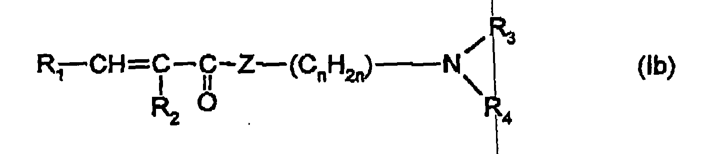
dans lesquelles. R₁ et R₂, identiques ou différents, représentent un atome d'hydrogène ou un radical méthyle, R₃, R₄ et R₅, identiques ou différentes, représente un radical alkyle linéaire ou ramifié ayant de 1 à 30 atomes de carbone,
Z représente un groupe NH ou un atome d'oxygène,
n est un nombre entier de 2 à 5,
A⁻ est un anion issu d'un acide organique ou minéral;
2) an moins un monomètre de formule (II)
R₆-CH= CR₇-COOH (II)
dans laquelle, R₆ et R₇, identiques ou différents, représentent un atome d'hydrogène ou un radical méthyle;
et
3) au moins un monomère de formule (III) :
R₆- CH=CR₇-COXR₈ (III)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (Ia), (Ib) ou (III) comportant au moins une chaîne grasse.

39. Composition selon la revendication 38, **caractérisée en ce que** les monomères de formule (Ia) et (Ib) sont choisis dans le groupe constitué par le diméthylaminoéthylméthacrylate, le diméthylaminoéthylacrylate, le diéthylaminoéthylméthacrylate, le diéthylaminoéthylacrylate, le diméthylaminopropylméthacrylate, le diméthylaminopropylacrylate, le diméthylaminopropylméthacrylamide, le diméthylaminopropylacrylamide, éventuellement quaternisés.

40. Composition selon la revendication 33 ou 39, **caractérisée en en ce que** le monomère de formule (Ia) est choisi parmi le chlorure d'acrylamidopropyl triméthyl ammonium et le chlorure de méthacrylamidopropyl triméthyl ammonium.

41. Composition selon la revendication 38, **caractérisée en ce que** le monomère de formule (II) choisi dans le groupe constitué par l'acide acrylique, l'acide méthacrylique, l'acide crotonique et l'acide méthyl-2 crotonique.

42. Composition selon la revendication 38, **caractérisée en ce que** le monomère de formule (III) est choisi dans le groupe constitué par les acrylates ou méthacrylates d'alkyle en C₁₂-C₂₂, et de préférence en C₁₆-C₁₈.

43. Composition selon l'une quelconque des revendications précédentes, **caractérisée en ce que** le ou les polymères associatifs sont présents dans la composition à des teneurs en poids comprises entre 0,05 et 10%, et de préférence entre 0,1 et 5% du poids total de la composition.

44. Composition selon l'une quelconque des revendications 1 à 20, **caractérisée en ce que** le polymère associatif est un polymère à chaîne grasse de type cationique.

45. Composition selon la revendication 44, **caractérisée en ce que** le polymère associatif est choisi parmi les polyuréthannes cationiques.

46. Composition selon l'un quelconque des revendications précédentes, **caractérisée en ce que** le rapport en poids, de l'amide d'une alcanolamine et d'un acide gras en C₁₄-C₃₀ sur l'alcool gras non oxyéthyléné est compris entre 0,1 et 10, de préférence entre 0,5 et 5.

47. Composition selon l'une quelconque des revendications précédentes, **caractérisée en ce que** la composition contient en outre au moins un polymère substantif amphotère ou cationique différent de ceux définis dans l'une quelconque des revendications 20 à 44.

48. Composition selon la revendication 47, **caractérisée en ce que** le polymère substantif est l'homopolymère de chlorure de diméthyldiallylammonium.

49. Composition selon la revendication 47, **caractérisée en ce que** le polymère substantif est un polymère aux motifs récurrents de formule (W) suivante :

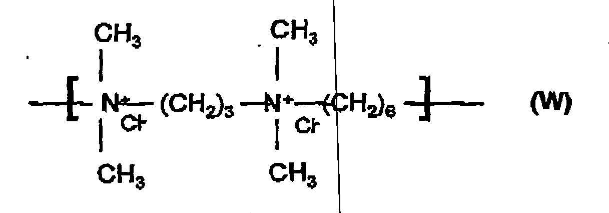

50. Composition selon la revendication 47, **caractérisée en ce que** le polymère substantif est un polymère aux motifs récurrents de formule (U) suivante :

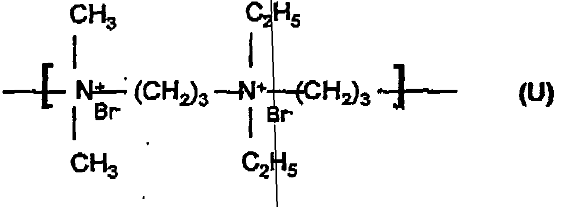

51. Composition selon l'une quelconque des revendications 47 à 50, **caractérisée en ce que** le ou les polymères substantifs cationiques ou amphotères représentent de 0,01 à 10%, de préférence de 0,05 à 5%, et encore de préférence de 0,1 à 3% du poids total de la composition.

52. Composition selon l'une quelconque des revendications précédentes, **caractérisée en ce qu'**elle comprend au moins un tensioactif choisi parmi les tensioactifs anioniques, amphotères, non ioniques, zwitterioniques et cationiques.

53. Composition selon la revendication 52, **caractérisée en ce que** les tensioactifs représentent de 0,01 à 40% et de préférence de 0,5 à 30% en poids, du poids total de la composition.

54. Composition selon l'une quelconque des revendications précédentes, **caractérisée en ce qu'**elle comprend au moins un épaississant d'appoint.

55. Composition selon la revendication 54, **caractérisée en ce que** l'épaississant d'appoint est un épaississant cellulosique, un dérivé de gomme de guar, une gomme d'origine microbienne, un épaississant synthétique.

56. Composition selon la revendication 54 on 55, **caractérisée en ce que** le ou les épaississants d'appoint représentent de 0,01 à 10% en poids du poids total de la composition.

57. Composition selon l'une quelconque des revendications précédentes, **caractérisée en ce qu'**elle comprend en outre au moins un agent réducteur, dans des quantités allant de 0,05 à 1,5% en poids par rapport au poids total de la composition.

58. Composition prête à l'emploi selon l'une quelconque des revendications précédentes, **caractérisée en ce qu'**elle comprend en outre un agent oxydant.

59. Composition selon la revendication 58, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

60. Composition selon la revendication 59, **caractérisée en ce que** l'agent oxydant est le peroxyde d'hydrogène.

61. Composition selon la revendication 60, **caractérisée en ce que** l'agent oxydant est une solution d'eau oxygénée dont le titre varie de 1 à 40 volumes.

62. Composition selon la revendication 57, **caractérisée en ce qu'**elle possède un pH allant de 4 à 11.

## Claims

1. Composition for the oxidation dyeing of keratin fibres, in particular of human keratin fibres, such as the hair, **characterized in that** it comprises, in a medium that is suitable for dyeing,
a) at least one oxidation dye,
b) at least one non-oxyalkylenated fatty alcohol,
c) at least one associative polymer chosen from amphiphilic polymers, and
d) at least one amide of an alkanolamine and a C₁₄-C₃₀ fatty acid, the ratio by weight of the amide of an alkanolamine and a C₁₄-C₃₀ fatty acid to the associative polymer being between 5 and 20.

2. Composition according to Claim 1, **characterized in that** the amide of an alkanolamine and a C₁₄-C₃₀ fatty acid is chosen from the amides of a C₂-C₁₀ alkanolamine and a C₁₄-C₃ₒ fatty acid and preferably from the amides of a C₂-C₁₀ alkanolamine and a C₁₄-C₂₂ fatty acid.

3. Composition according to Claim 1 or 2, **characterized in that** the amide of an alkanolamine and a C₁₄-C₃₀ fatty acid is chosen from
- oleic acid diethanolamide,
- myristic acid monoethanolamide,
- soya fatty acids diethanolamide,
- stearic acid ethanolamide,
- oleic acid monoisopropanolamide,
- linoleic acid diethanolamide,
- stearic acid monoethanolamide,
- behenic acid monoethanolamide,
- isostearic acid monoisopropanolamide,
- erucic acid diethanolamide, and
- ricinoleic acid monoethanolamide.

4. Composition according to Claim 3, **characterized in that** the amide of an alkanolamine and a C₁₄-C₃₀ fatty acid is chosen from
- oleic acid diethanolamide,
- myristic acid monoethanolamide,
- soya fatty acids diethanolamide,
- stearic acid ethanolamide,
- linoleic acid diethanolamide,
- stearic acid monoethanolamide,
- behenic acid monoethanolamide,
- erucic acid diethanolamide, and
- ricinoleic acid monoethanolamide.

5. Composition according to Claim 1 or 2, **characterized in that** the amide of an alkanolamine and a C₁₄-C₃₀ fatty acid is present in the composition in proportions by weight of between 0.1% and 10% and preferably between 1% and 5% of the total weight of the composition.

6. Composition according to any one of the preceding claims, **characterized in that** the non-oxyalkylenated fatty alcohol is chosen from lauryl, cetyl, stearyl, oleyl, behenyl, linoleyl, undecylenyl, palmitoleyl, arachidonyl and erucyl alcohols and mixtures thereof.

7. Composition according to any one of the preceding claims, **characterized in that** the non-oxyalkylenated fatty alcohol is present in the composition in proportions by weight of between 0.1% and 20% and preferably between 1% and 10% of the total weight of the composition.

8. Composition according to any one of the preceding claims, **characterized in that** the oxidation dye is chosen from oxidation bases and/or couplers.

9. Composition according to Claim 8, **characterized in that** it comprises at least one oxidation base.

10. Composition according to Claim 8 or 9, **characterized in that** the oxidation bases are chosen from ortho- and para-phenylenediamines, double bases, ortho- and para-aminophenols, and heterocyclic bases, and also the addition salts of these compounds with an acid.

11. Composition according to Claim 10, **characterized in that** the para-phenylenediamines are chosen from the compounds of formula (I) below: 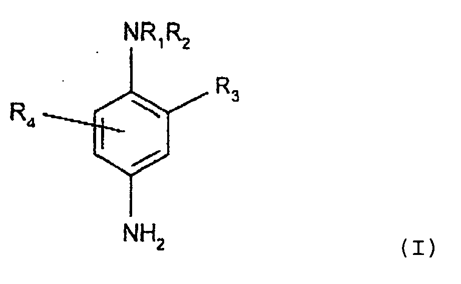 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous, phenyl or 4'-aminophenyl group;
- R₂ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous group;
R₁ and R₂ may also form, with the nitrogen atom that bears them, a 5- or 6-membered nitrogen heterocycle optionally substituted with one or more alkyl, hydroxyl or ureido groups;
- R₃ represents a hydrogen atom, a halogen atom, a C₁-C₄ alkyl radical, a sulpho radical, a carboxyl radical, a C₁-C₄ monohydroxyalkyl radical, a C₁-C₄ hydroxyalkoxy radical, an acetylamino(C₁-C₄)alkoxy radical, a mesylamino(C₁-C₄)alkoxy radical or a carbamoylamino(C₁-C₄)alkoxy radical,
- R₄ represents a hydrogen or halogen atom or a C₁-C₄ alkyl radical.

12. Composition according to Claim 10, **characterized in that** the double bases are chosen from the compounds of formula (II) below: 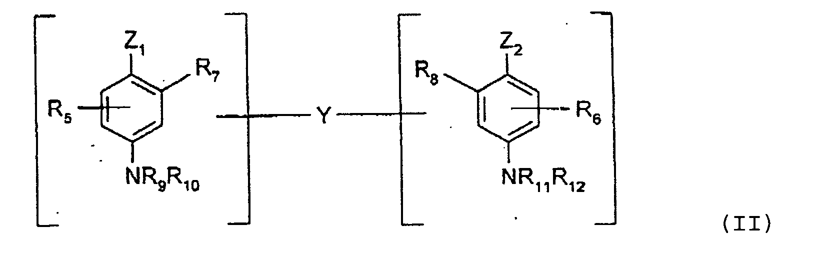 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more heteroatoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

13. Composition according to Claim 10, **characterized in that** the para-aminophenols are chosen from the compounds of formula (III) below: 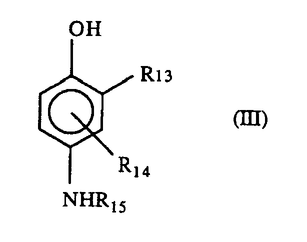 in which:
R₁₃ represents a hydrogen atom, a halogen atom, or a C₁-C₄ alkyl, C₁-C₄ monohydroxyalkyl, (C₁-C₄)alkoxy(C₁-C₄)-alkyl, C₁-C₄ aminoalkyl or hydroxy(C₁-C₄)alkylamino-(C₁-C₄) alkyl radical,
R₁₄ represents a hydrogen atom, a halogen atom, or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄)alkyl radical.
R₁₅ represents a hydrogen atom or a C₁-C₄ alkyl radical.

14. Composition according to Claim 10, **characterized in that** the heterocyclic bases are chosen from pyridine derivatives, pyrimidine derivatives, and pyrazole derivatives.

15. Composition according to any one of Claims 8 to 14, **characterized in that** the oxidation bases represent from 0.0005% to 12% and preferably from 0.005% to 8% by weight relative to the total weight of the composition.

16. Composition according to Claim 8, **characterized in that** the couplers are chosen from meta-phenylenediamines, meta-aminophenols, metadiphenols and heterocyclic couplers, and the addition salts of these compounds with an acid.

17. Composition according to Claim 8 or 16, **characterized in that** the couplers represent from 0.0001% to 10% and preferably from 0.005% to 5% by weight relative to the total weight of the composition.

18. Composition according to any one of Claims 8 to 17, **characterized in that** the addition salts with an acid of the oxidation dyes are chosen from hydrochlorides, hydrobromides, sulphates, tartrates, lactates and acetates.

19. Composition according to any one of the preceding claims, **characterized in that** it also comprises direct dyes.

20. Composition according to any one of the preceding claims, **characterized in that** the associative amphiphilic polymer is chosen from non-ionic, anionic, cationic and amphoteric associative polymers.

21. Composition according to Claim 20, **characterized in that** the associative polymer is a fatty-chain anionic associative polymer comprising at least one hydrophilic unit and at least one fatty-chain allyl ether unit.

22. Composition according to Claim 21, **characterized in that** the hydrophilic unit is an ethylenic unsaturated anionic monomer and preferably a vinylcarboxylic acid.

23. Composition according to Claim 21, **characterized in that** the fatty-chain allyl ether unit is a monomer of formula (I) below:
CH₂ = C R' CH₂ O Bₙ R (I)
in which R' denotes H or CH₃, B denotes an ethyleneoxy radical, n is zero or denotes an integer ranging from 1 to 100, R denotes a hydrocarbon-based radical chosen from alkyl, arylalkyl, aryl, alkylaryl and cycloalkyl radicals, containing from 8 to 30 carbon atoms, preferably 10 to 24 carbon atoms and even more particularly from 12 to 18 carbon atoms.

24. Composition according to Claim 21, **characterized in that** the fatty-chain anionic associative polymer comprises at least one hydrophilic unit of unsaturated olefinic carboxylic acid type and at least one hydrophobic unit of unsaturated carboxylic acid (C₁₀-C₃₀) alkyl ester type.

25. Composition according to Claim 24, **characterized in that** the hydrophilic unit of unsaturated olefinic carboxylic acid type corresponds to the monomer of formula (II) below: 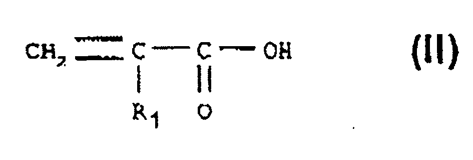 in which R₁ denotes H or CH₃ or C₂H₅, and in which the hydrophobic unit of unsaturated carboxylic acid (C₁₀-C₃₀) alkyl ester type corresponds to the monomer of formula (III) below: 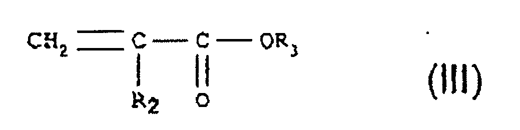 in which R₂ denotes H or CH₃ or C₂H₅, R₃ denoting a C₁₀-C₃₀ and preferably C₁₂-C₂₂ alkyl radical.

26. Composition according to Claim 21, **characterized in that** the fatty-chain associative polymer is a maleic anhydride/C₃₀-C₃₈ α-olefin/alkyl maleate terpolymer.

27. Composition according to Claim 21, **characterized in that** the fatty-chain associative polymer is an acrylic terpolymer comprising:
(a) about 20% to 70% by weight of a carboxylic acid containing α,β-monoethylenic unsaturation,
(b) about 20% to 80% by weight of a non-surfactant monomer containing α,β-monoethylenic unsaturation and being other than (a),
(c) about 0.5% to 60% by weight of a non-ionic monourethane which is the product of reaction of a monohydric surfactant with a monoisocyanate containing monoethylenic unsaturation.

28. Composition according to Claim 21, **characterized in that** the fatty-chain associative polymer is chosen from copolymers comprising among their monomers a carboxylic acid containing α,β-monoethylenic unsaturation and an ester of carboxylic acid containing α,β-monoethylenic unsaturation and of an oxyalkylenated fatty alcohol.

29. Composition according to Claim 20, **characterized in that** the non-ionic fatty-chain associative polymer is chosen from the group consisting of:
(1) celluloses modified with groups comprising at least one fatty chain;
(2) hydroxypropylguars modified with groups comprising at least one fatty chain;
(3) polyurethane polyethers comprising in their chain both hydrophilic blocks of polyoxyethylenated kind and hydrophobic blocks which are aliphatic sequences alone and/or cycloaliphatic and/or aromatic sequences;
(4) copolymers of vinylpyrrolidone and of fatty-chain hydrophobic monomers;
(5) copolymers of C₁-C₆ alkyl methacrylates or acrylates and of amphiphilic monomers comprising at least one fatty chain;
(6) copolymers of hydrophilic methacrylates or acrylates and of hydrophobic monomers comprising at least one fatty chain;
(7) polymers with an aminoplast ether skeleton containing at least one fatty chain.

30. Composition according to Claim 29, **characterized in that** the polyurethane polyethers comprise at least two hydrocarbon-based lipophilic chains having from 8 to 30 carbon atoms, separated by a hydrophilic block, the hydrocarbon-based chains being pendent chains or chains at the end of the hydrophilic block.

31. Composition according to Claim 29, **characterized in that** the polyurethane polyethers are multiblock, and preferably in triblock form.

32. Composition according to Claim 20, **characterized in that** the associative polymer is a cationic polymer comprising at least one fatty chain and chosen from:
(i) quaternized celluloses modified with groups comprising at least one fatty chain,
(ii) quaternized hydroxyethylcelluloses modified with groups comprising at least one fatty chain,
(iii) cationic polyurethanes,
(iv) cationic polyvinyllactams,
(v) acrylic terpolymer consisting of acrylates, amino (meth) acrylates and C₁₀-C₃₀ alkyl itaconate, polyoxyethylenated with 20 mol of ethylene oxide.

33. Composition according to Claim 32, **characterized in that** the alkyl groups of the quaternized celluloses or hydroxyethylcelluloses contain from 8 to 30 carbon atoms.

34. Composition according to Claim 32 or 33, **characterized in that** the cationic amphiphilic polymer is a quaternized hydroxyethylcellulose modified with a C₁₂ or C₁₈ alkyl group.

35. Composition according to Claim 32, **characterized in that** the cationic amphiphilic polyurethane is a polymer of formula (Ia) below:
R-X-(P)ₙ-[L-(Y)ₘ]ᵣ-L'-(P')ₚ-X'-R' (Ia)
in which:
R and R', which may be identical or different, represent a hydrophobic group or a hydrogen atom;
X and X', which may be identical or different, represent a group comprising an amine function optionally bearing a hydrophobic group, or alternatively the group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,
n, m and p each amount, independently of each other, to between 0 and 1000;
the molecule containing at least one protonated or quaternized amine function and at least one hydrophobic group.

36. Composition according to Claim 20, **characterized in that** the amphoteric polymer comprises at least one fatty chain having 8 to 30 carbon atoms and at least one non-cyclic cationic unit.

37. Composition according to Claim 36, **characterized in that** the amphoteric polymer contains from 1 to 20 mol% of monomer comprising a fatty chain, relative to the total number of moles of monomers.

38. Composition according to Claim 36 or 37, **characterized in that** the amphoteric polymers comprise:
1) at least one monomer of formula (Ia) or (Ib): 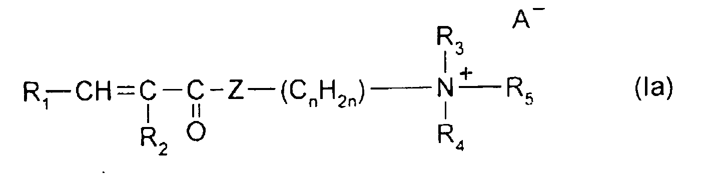 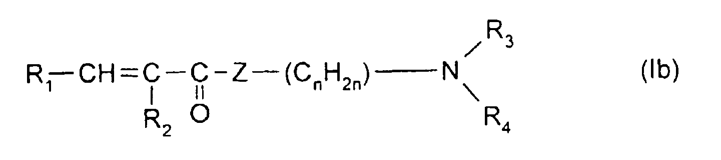
in which R₁ and R₂, which may be identical or different, represent a hydrogen atom or a methyl radical, R₃, R₄ and R₅, which may be identical or different, represent a linear or branched alkyl radical containing from 1 to 30 carbon atoms,
Z represents an NH group or an oxygen atom,
n is an integer from 2 to 5,
A⁻ is an anion derived from an organic or mineral acid;
2) at least one monomer of formula (II)
R₆-CH = CR₇-COOH (II)
in which R₆ and R₇, which may be identical or different, represent a hydrogen atom or a methyl radical;
and
3) at least one monomer of formula (III):
R₆-CH= CR₇- COXR₈ (III)
in which R₆ and R₇, which may be identical or different, represent a hydrogen atom or a methyl radical, X denotes an oxygen or nitrogen atom and R₈ denotes a linear or branched alkyl radical containing from 1 to 30 carbon atoms;
at least one of the monomers of formula (Ia), (Ib) or (III) comprising at least one fatty chain.

39. Composition according to Claim 38, **characterized in that** the monomers of formulae (Ia) and (Ib) are chosen from the group consisting of dimethylaminoethyl methacrylate, dimethylaminoethyl acrylate, diethylaminoethyl methacrylate, diethylaminoethyl acrylate, dimethylaminopropyl methacrylate, dimethylaminopropyl acrylate, dimethylaminopropylmethacrylamide, dimethylaminopropylacrylamide, which are optionally quaternized.

40. Composition according to Claim 38 or 39, **characterized in that** the monomer of formula (Ia) is chosen from acrylamidopropyltrimethylammonium chloride and methacrylamidopropyltrimethylammonium chloride.

41. Composition according to Claim 38, **characterized in that** the monomer of formula (II) is chosen from the group consisting of acrylic acid, methacrylic acid, crotonic acid and 2-methylcrotonic acid.

42. Composition according to Claim 38, **characterized in that** the monomer of formula (III) is chosen from the group consisting of C₁₂-C₂₂ and preferably C₁₆-C₁₈ alkyl acrylates or methacrylates.

43. Composition according to any one of the preceding claims, **characterized in that** the associative polymer or polymers are present in the composition in amounts by weight of between 0.05% and 10% and preferably between 0.1% and 5% of the total weight of the composition.

44. Composition according to any one of Claims 1 to 20, **characterized in that** the associative polymer is a fatty-chain polymer of cationic type.

45. Composition according to Claim 44, **characterized in that** the associative polymer is chosen from cationic polyurethanes.

46. Composition according to any one of the preceding claims, **characterized in that** the ratio by weight of the amide of an alkanolamine and a C₁₄-C₃₀ fatty acid to the non-oxyethylenated fatty alcohol is between 0.1 and 10, preferably between 0.5 and 5.

47. Composition according to any one of the preceding claims, **characterized in that** the composition further comprises at least one amphoteric or cationic substantive polymer different from those defined in any one of Claims 20 to 44.

48. Composition according to Claim 47, **characterized in that** the substantive polymer is the homopolymer of dimethyldiallylammonium chloride.

49. Composition according to Claim 47, **characterized in that** the substantive polymer is a polymer comprising repeating units of formula (W) below:

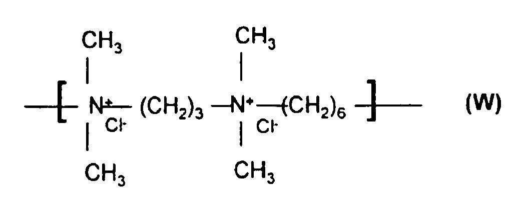

50. Composition according to Claim 47, **characterized in that** the substantive polymer is a polymer comprising repeating units of formula (U) below:

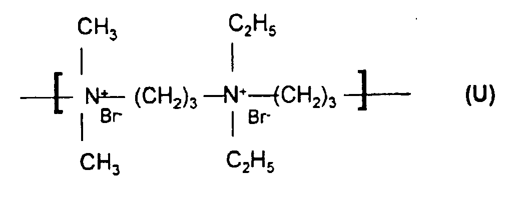

51. Composition according to any one of Claims 47 to 50, **characterized in that** the cationic or amphoteric substantive polymer or polymers represent from 0.01% to 10%, preferably from 0.05% to 5% and even more preferably from 0.1% to 3% of the total weight of the composition.

52. Composition according to any one of the preceding claims, **characterized in that** it comprises at least one surfactant chosen from anionic, amphoteric, non-ionic, zwitterionic and cationic surfactants.

53. Composition according to Claim 52, **characterized in that** the surfactants represent from 0.01% to 40% and preferably from 0.5% to 30% by weight relative to the total weight of the composition.

54. Composition according to any one of the preceding claims, **characterized in that** it comprises at least one supplementary thickener.

55. Composition according to Claim 54, **characterized in that** the supplementary thickener is a cellulosic thickener, a guar gum derivative, a gum of microbial origin or a synthetic thickener.

56. Composition according to Claim 54 or 55, **characterized in that** the supplementary thickener or thickeners represent from 0.01% to 10% by weight relative to the total weight of the composition.

57. Composition according to any one of the preceding claims, **characterized in that** it also comprises at least one reducing agent, in amounts ranging from 0.05% to 1.5% by weight relative to the total weight of the composition.

58. Ready-to-use composition according to any one of the preceding claims, **characterized in that** it also comprises an oxidizing agent.

59. Composition according to Claim 58, **characterized in that** the oxidizing agent is chosen from hydrogen peroxide, urea peroxide, alkali metal bromates or ferricyanides, persalts, and redox enzymes together where appropriate with the respective donor or co-factor thereof.

60. Composition according to Claim 59, **characterized in that** the oxidizing agent is hydrogen peroxide.

61. Composition according to Claim 60, **characterized in that** the oxidizing agent is an aqueous hydrogen peroxide solution whose titre ranges from 1 to 40 volumes.

62. Composition according to Claim 57, **characterized in that** it has a pH ranging from 4 to 11.

## Patentansprüche

1. Zusammensetzung zum Oxidationsfärben von Keratinfasern, insbesondere menschlichen Keratinfasern, wie Haaren, **dadurch gekennzeichnet, dass** sie in einem für die Färbung geeigneten Medium
a) mindestens einen Oxidationsfarbstoff,
b) mindestens einen nicht oxyalkenylierten Fettalkohol,
c) mindestens ein Assoziativpolymer, das unter amphiphilen Polymeren ausgewählt ist, und
d) mindestens ein Amid eines Alkanolamins und einer C₁₄-C₃₀-Fettsäure
umfasst; wobei das Gewichtsverhältnis von Amid eines Alkanolamins und einer C₁₄-C₃₀-Fettsäure zu Assoziativpolymer zwischen 5 und 20 liegt.

2. Zusammensetzung nach Anspruch 1, **dadurch gekennzeichnet, dass** das Amid eines Alkanolamins und einer C₁₄-C₃₀-Fettsäure unter den Amiden eines C₂-C₁₀-Alkanolamins und einer C₁₄-C₃₀-Fettsäure und vorzugsweise unter den Amiden eines C₂-C₁₀-Alkanolamins und einer C₁₄-C₂₂-Fettsäure ausgewählt ist.

3. Zusammensetzung nach Anspruch 1 oder 2, **dadurch gekennzeichnet, dass** das Amid eines Alkanolamins und einer C₁₄-C₃₀-Fettsäure unter
- Ölsäurediethanolamid,
- Myristinsäuremonoethanolamid,
- Sojafettsäurediethanolamid,
- Stearinsäureethanolamid,
- Ölsäuremonoisopropanolamid,
- Linölsäurediethanolamid,
- Stearinsäuremonoethanolamid,
- Behensäuremonoethanolamid,
- Isostearinsäuremonoisopropanolamid,
- Erucasäurediethanolamid und
- Ricinolsäuremonoethanolamid
ausgewählt ist.

4. Zusammensetzung nach Anspruch 3, **dadurch gekennzeichnet, dass** das Amid eines Alkanolamins und einer C₁₄-C₃₀-Fettsäure unter
- Ölsäurediethanolamid,
- Myristinsäuremonoethanolamid,
- Sojafettsäurediethanolamid,
- Stearinsäureethanolamid,
- Linölsäurediethanolamid,
- Stearinsäuremonoethanolamid,
- Behensäuremonoethanolamid,
- Erucasäurediethanolamid und
- Ricinolsäuremonoethanolamid
ausgewählt ist.

5. Zusammensetzung nach Anspruch 1 oder 2, **dadurch gekennzeichnet, dass** das Amid eines Alkanolamins und einer C₁₄-C₃₀-Fettsäure in der Zusammensetzung in Gewichtsanteilen zwischen 0,1 und 10% und vorzugsweise zwischen 1 und 5%, bezogen auf das Gesamtgewicht der Zusammensetzung, vorliegt.

6. Zusammensetzung nach einem der vorhergehenden Ansprüche, **dadurch gekennzeichnet, dass** der nicht oxyalkenylierte Fettalkohol unter Laurylalkohol, Cetylalkohol, Stearylalkohol, Oleylalkohol, Behenylalkohol, Linoleylalkohol, Undecylenylalkohol, Palmitoleylalkohol, Arachidonylalkohol und Erucylalkohol und Mischungen davon ausgewählt ist.

7. Zusammensetzung nach einem der vorhergehenden Ansprüche, **dadurch gekennzeichnet, dass** der nicht oxyalkenylierte Fettalkohol in der Zusammensetzung in Gewichtsanteilen zwischen 0,1 und 20% und vorzugsweise zwishen 1 und 10%, bezogen auf das Gesamtgewicht der Zusammensetzung, vorliegt.

8. Zusammensetzung nach einem der vorhergehenden Ansprüche, **dadurch gekennzeichnet, dass** der Oxidationsfarbstoff unter Oxidationsbasen und/oder Kupplern ausgewählt ist.

9. Zusammensetzung nach Anspruch 8, **dadurch gekennzeichnet, dass** sie mindestens eine Oxidationsbase umfasst.

10. Zusammensetzung nach Anspruch 8 oder 9, **dadurch gekennzeichnet, dass** die Oxidationsbasen unter ortho- und para-Phenylendiaminen, Doppelbasen, ortho- und para-Aminophenolen, heterocyclischen Basen und Additionssalzen dieser Verbindungen mit einer Säure ausgewählt sind.

11. Zusammensetzung nach Anspruch 10, **dadurch gekennzeichnet, dass** die para-Phenylendiamine unter den Verbindungen der nachstehenden Formel (I) ausgewählt sind: 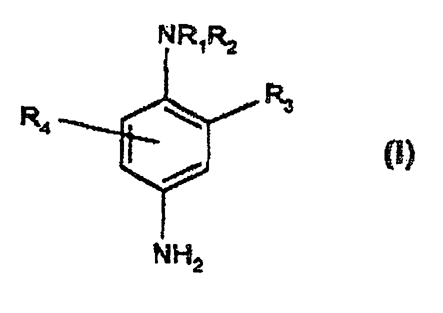 worin:
- R₁ für ein Wasserstoffatom, einen C₁-C₄-Alkylrest, einen C₁-C₄-Monohydroxyalkylrest, einen C₂-C₄-Polyhydroxyalkylrest, einen (C₁-C₄) -Alkoxy-(C₁-C₄) -alkylrest oder einen C₁-C₄-Alkylrest, der durch eine stickstoffhaltige Gruppe, eine Phenylgruppe oder eine 4'-Aminophenylgruppe substituiert ist, steht;
- R₂ für ein Wasserstoffatom, einen C₁-C₄-Alkylrest, einen C₁-C₄-Monohydroxyalkylrest, einen C₂-C₄-Polyhydroxyalkylrest, einen (C₁-C₄) -Alkoxy-(C₁-C₄)-alkylrest oder einen C₁-C₄-Alkylrest, der durch eine stickstoffhaltige Gruppe substituiert ist, steht;
R₁ und R₂ auch mit dem Stickstoffatom, an das sie gebunden sind, einen 5- oder 6-gliedrigen stickstoffhaltigen Heterocyclus, der gegebenenfalls durch eine oder mehrere Alkyl-, Hydroxyl- oder Ureidogruppen substituiert ist, bilden können;
- R₃ für ein Wasserstoffatom, ein Halogenatom, einen C₁-C₄-Alkylrest, einen Sulforest, einen Carboxylrest, einen C₁-C₄-Monohydroxyalkylrest, einen C₁-C₄-Hydroxyalkoxyrest, einen Acetylamino-C₁-C₄-alkoxyrest, einen Mesylamino-C₁-C₄-alkoxyrest oder einen Carbamoylamino-C₁-C₄-alkoxyrest steht,
- R₄ für ein Wasserstoffatom, ein Halogenatom oder einen C₁-C₄-Alkylrest steht.

12. Zusammensetzung nach Anspruch 10, **dadurch gekennzeichnet, dass** die Doppelbasen unter den Verbindungen der nachstehenden Formel (II) ausgewählt sind: 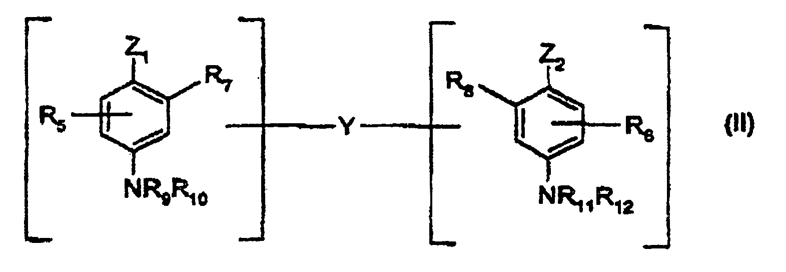 worin:
- Z₁ und Z₂ gleich oder verschieden sind und für einen Hydroxyl- oder -NH₂-Rest, der durch einen C₁-C₄-Rest oder durch einen Linkerarm Y substituiert sein kann, stehen;
- der Linkerarm Y für eine lineare oder verzweigte Alkylenkette mit 1 bis 14 Kohlenstoffatomen, die durch eine oder mehrere stickstoffhaltige Gruppen und/oder durch ein oder mehrere Heteroatome unterbrochen oder terminiert und gegebenenfalls durch einen oder mehrere Hydroxyl- oder C₁-C₆-Alkoxyeste substituiert sein kann, steht;
- R₅ und R₆ für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest oder einen Linkerarm Y stehen;
- R₇, R₈, R₉, R₁₀, R₁₁ und R₁₂ gleich oder verschieden sind und für ein Wasserstoffatom, einen Linkerarm Y oder einen C₁-C₄-Alkylrest stehen;
mit der Maßgabe, dass die Verbindungen der Formel (II) nur einen Linkerarm Y pro Molekül enthalten.

13. Zusammensetzung nach Anspruch 10, **dadurch gekennzeichnet, dass** die para-Aminophenole unter den Verbindungen der nachstehenden Formel (III) ausgewählt sind: 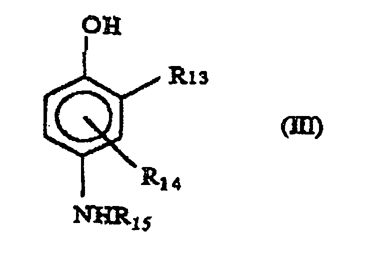 worin:
R₁₃ für ein Wasserstoffatom, ein Halogenatom, einen C₁-C₄-Alkylrest, einen C₁-C₄-Monohydroxyalkylrest, einen (C₁-C₄) -Alkoxy- (C₁-C₄) -alkylrest, einen C₁-C₄-Aminoalkylrest oder einen Hydroxy- (C₁-C₄) -amino-C₁-C₄-alkylrest steht,
R₁₄ für ein Wasserstoffatom, ein Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest, einen C₁-C₄-Cyanoalkylrest oder einen (C₁-C₄)-Alkoxy-(C₁-C₄)-alkylrest steht,
R₁₅ für ein Wasserstoffatom oder einen C₁-C₄-Alkylrest steht.

14. Zusammensetzung nach Anspruch 10, **dadurch gekennzeichnet, dass** die heterocyclischen Basen unter Pyridinderivaten, Pyrimidinderivaten und Pyrazolderivaten ausgewählt sind.

15. Zusammensetzung nach einem der Ansprüche 8 bis 14, **dadurch gekennzeichnet, dass** die Oxidationsbasen 0,0005 bis 12 Gew.-% und vorzugsweise 0,005 bis 8 Gew.-% des Gesamtgewichts der Zusammensetzung ausmachen.

16. Zusammensetzung nach Anspruch 8, **dadurch gekennzeichnet, dass** die Kuppler unter meta-Phenylendiaminen, meta-Aminophenolen, meta-Diphenolen, heterocyclischen Kupplern und Additionssalzen dieser Verbindungen mit einer Säure ausgewählt sind.

17. Zusammensetzung nach Anspruch 8 oder 16, **dadurch gekennzeichnet, dass** die Kuppler 0,0001 bis 10 Gew.-% und vorzugsweise 0,005 bis 5 Gew.-% des Gesamtgewichts der Zusammensetzung ausmachen.

18. Zusammensetzung nach einem der Ansprüche 8 bis 17, **dadurch gekennzeichnet, dass** die Additionssalze der Oxidationsfarbstoffe mit einer Säure unter Hydrochloriden, Hydrobromiden, Sulfaten, Tartraten, Lactaten und Acetaten ausgewählt sind.

19. Zusammensetzung nach einem der vorhergehenden Ansprüche, **dadurch gekennzeichnet, dass** sie außerdem Direktfarbstoffe umfasst.

20. Zusammensetzung nach einem der vorhergehenden Ansprüche, **dadurch gekennzeichnet, dass** das amphiphile Assoziativpolymer unter nichtionischen, anionischen, kationischen und amphoteren Assoziativpolymeren ausgewählt ist.

21. Zusammensetzung nach Anspruch 20, **dadurch gekennzeichnet, dass** es sich bei dem Assoziativpolymer um ein anionisches Fettkettenassoziativpolymer mit mindestens einer hydrophilen Einheit und mindestens einer Fettkettenallylether-Einheit handelt.

22. Zusammensetzung nach Anspruch 21, **dadurch gekennzeichnet, dass** es sich bei der hydrophilen Einheit um ein ethylenisch ungesättigtes anionisches Monomer und vorzugsweise eine Vinylcarbonsäure handelt.

23. Zusammensetzung nach Anspruch 21, **dadurch gekennzeichnet, dass** es sich bei der Fettkettenallylether-Einheit um ein Monomer der folgenden Formel (I) handelt:
CH₂ = C R' CH₂ O Bₙ R (I)
worin R' für H oder CH₃ steht, B für einen Ethylenoxyrest steht, n gleich null ist oder für eine ganze Zahl von 1 bis 100 steht, R für einen unter Alkyl-, Arylalkyl-, Aryl-, Alkylaryl- und Cycloalkylresten ausgewählten Kohlenwasserstoffrest mit 8 bis 30 Kohlenstoffatomen, vorzugsweise 10 bis 24 Kohlenstoffatomen und noch spezieller 12 bis 18 Kohlenstoffatomen steht.

24. Zusammensetzung nach Anspruch 21, **dadurch gekennzeichnet, dass** das anionische Fettkettenassoziativpolymer mindestens eine hydrophile Einheit vom Typ olefinisch ungesättigte Carbonsäure und mindestens eine hydrophobe Einheit vom Typ ungesättigter Carbonsäure-(C₁-C₃₀)-alkylester umfasst.

25. Zusammensetzung nach Anspruch 24, **dadurch gekennzeichnet, dass** die hydrophile Einheit vom Typ olefinisch ungesättigte Carbonsäure dem Monomer der nachstehenden Formel (II) entspricht: 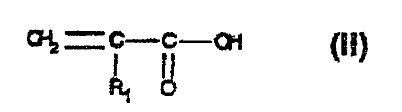 worin R₁ für H oder CH₃ oder C₂H₅ steht, und die hydrophobe Einheit vom Typ ungesättigter Carbonsäure-(C₁₀-C₃₀)-alkylester dem Monomer der nachstehenden Formel (III) entspricht: 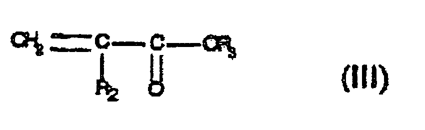 worin R₂ für H oder CH₃ oder C₂H₅ steht und R₃ für einen C₁₀-C₃₀-Alkylrest und vorzugsweise einen C₁₂-C₂₂-Alkylrest steht.

26. Zusammensetzung nach Anspruch 21, **dadurch gekennzeichnet, dass** es sich bei dem FettkettenAssoziativpolymer um ein Terpolymer von Maleinsäureanhydrid, C₃₀-₃₈-α-Olefin und Alkylmaleat handelt.

27. Zusammensetzung nach Anspruch 21, **dadurch gekennzeichnet, dass** es sich bei dem Fettketten-Assoziativpolymer um ein Acrylterpolymer, umfassend:
(a) etwa 20 bis 70 Gew.-% einer α,β-monoethylenisch ungesättigten Carbonsäure,
(b) etwa 20 bis 80 Gew.-% eines nichttensidischen α,β-monoethylenisch ungesättigten Monomers, das von (a) verschieden ist,
(c) etwa 0,5 bis 60 Gew.-% eines nichtionischen Monourethans, bei dem es sich um das Produkt der Reaktion eines Monohydroxytensids mit einem monoethylenisch ungesättigten Monoisocyanat handelt, handelt.

28. Zusammensetzung nach Anspruch 21, **dadurch gekennzeichnet, dass** das Fettketten-Assoziativpolymer unter Copolymeren, die unter ihren Monomeren eine α,β-monoethylenisch ungesättigte Carbonsäure und einen α,β-monoethylenisch ungesättigten Carbonsäureester und einen oxyalkylierten Fettalkohol enthalten, ausgewählt ist.

29. Zusammensetzung nach Anspruch 20, **dadurch gekennzeichnet, dass** das nichtionische Fettketten-Assoziativpolymer aus der Gruppe bestehend aus:
(1) Cellulosen, die mit Gruppen mit mindestens einer Fettkette modifiziert sind;
(2) Hydroxypropylguars, die mit Gruppen mit mindestens einer Fettkette modifiziert sind;
(3) Polyurethanpolyethern, die in ihrer Kette sowohl hydrophile Blöcke vom Polyoxyethylen-Typ als auch hydrophobe Blöcke, bei denen es sich um aliphatische Ketten alleine und/oder cycloaliphatische und/oder aromatische Ketten handelt, enthalten;
(4) Copolymeren von Vinylpyrrolidon und hydrophoben Fettketten-Monomeren;
(5) Copolymeren von C₁-C₆-Alkylmethacrylaten oder -acrylaten und amphiphilen Monomeren mit mindestens einer Fettkette;
(6) Copolymeren von hydrophilen Methacrylaten oder Acrylaten und hydrophoben Monomeren mit mindestens einer Fettkette;
(7) Polymeren mit Aminoplastether-Gerüst mit mindestens einer Fettkette;
ausgewählt ist.

30. Zusammensetzung nach Anspruch 29, **dadurch gekennzeichnet, dass** die Polyetherpolyurethane mindestens zwei lipophile Kohlenwasserstoffketten mit 8 bis 30 Kohlenstoffatomen, die durch einen hydrophilen Block getrennt sind, enthalten, wobei es sich bei den Kohlenwasserstoffketten um Seitenketten oder Ketten am Ende des hydrophilen Blocks handelt.

31. Zusammensetzung nach Anspruch 29, **dadurch gekennzeichnet, dass** die Polyetherpolyurethane Multiblockpolymere sind und vorzugsweise in Triblockform vorliegen.

32. Zusammensetzung nach Anspruch 20, **dadurch gekennzeichnet, dass** das Assoziativpolymer ein kationisches Polymer mit mindestens einer Fettkette ist und unter:
(i) quaternisierten Cellulosen, die mit Gruppen mit mindestens einer Fettkette modifiziert sind,
(ii) quaternisierten Hydroxyethylcellulosen, die mit Gruppen mit mindestens einer Fettkette modifiziert sind,
(iii) kationischen Polyurethanen,
(iv) kationischen Polyvinyllactamen,
(v) Acrylterpolymer aus Acrylaten, Amino(meth)-acrylaten und mit 20 mol Ethylenoxid polyoxyethyleniertem C₁₀-C₃₀-Alkylitaconat,
ausgewählt ist.

33. Zusammensetzung nach Anspruch 32, **dadurch gekennzeichnet, dass** die Alkylgruppen der quaternisierten Cellulosen oder Hydroxyethylcellulosen 8 bis 30 Kohlenstoffatome enthalten.

34. Zusammensetzung nach Anspruch 32 oder 33, **dadurch gekennzeichnet, dass** es sich bei dem kationischen amphiphilen Polymer um eine quaternisierte Hydroxyethylcellulose, die durch eine C₁₂- oder C₁₈-Alkylgruppe modifiziert ist, handelt.

35. Zusammensetzung nach Anspruch 32, **dadurch gekennzeichnet, dass** es sich bei dem kationischen amphiphilen Polyurethan um ein Polymer der nachstehenden Formel (Ia) handelt:
R-X-(P)ₙ-[L-(Y)ₘ]ᵣ-L'-(P')ₚ-X'-R' (Ia)
worin:
R und R' gleich oder verschieden sind und für eine hydrophobe Gruppe oder ein Wasserstoffatom stehen;
X und X' gleich oder verschieden sind und für eine Gruppe mit einer Aminfunktion, die gegebenenfalls eine hydrophobe Gruppe trägt, oder auch die Gruppe L" stehen;
L, L' und L'' gleich oder verschieden sind und für eine von einem Diisocyanat abgeleitete Gruppe stehen;
P und P' gleich oder verschieden sind und für eine Gruppe mit einer Aminfunktion, die gegebenenfalls eine hydrophobe Gruppe trägt, stehen;
Y für eine hydrophile Gruppe steht;
r für eine ganze Zahl zwischen 1 und 100, vorzugsweise zwischen 1 und 50 und insbesondere zwischen 1 und 25 steht;
n, m und p jeweils unabhängig voneinander einen Wert zwischen 0 und 1000 aufweisen;
wobei das Molekül mindestens eine protonierte oder quaternisierte Aminfunktion und mindestens eine hydrophobe Gruppe enthält.

36. Zusammensetzung nach Anspruch 20, **dadurch gekennzeichnet, dass** das amphotere Polymer mindestens eine Fettkette mit 8 bis 30 Kohlenstoffatomen und mindestens eine nichtcyclische kationische Einheit enthält.

37. Zusammensetzung nach Anspruch 36, **dadurch gekennzeichnet, dass** das amphotere Polymer 1 bis 20 Mol-% Monomer mit einer Fettkette, bezogen auf die Gesamtzahl der Mole von Monomeren, enthält.

38. Zusammensetzung nach Anspruch 36 oder 37, **dadurch gekennzeichnet, dass** die amphoteren Polymere
1) mindestens ein Monomer der Formel (Ia) oder (Ib): 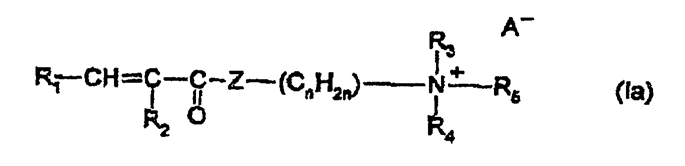 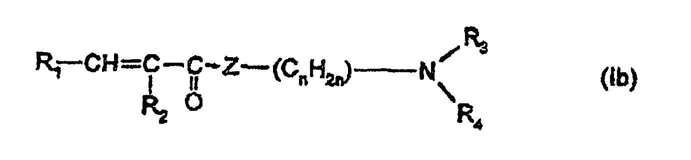
worin R₁ und R₂ gleich oder verschieden sind und für ein Wasserstoffatom oder einen Methylrest stehen, R₃, R₄ und R₅ gleich oder verschieden sind und für einen linearen oder verzweigten Alkylrest mit 1 bis 30 Kohlenstoffatomen stehen,
Z für eine NH-Gruppe oder ein Sauerstoffatom steht,
n für eine ganze Zahl von 2 bis 5 steht,
A⁻ für ein aus einer organischen oder anorganischen Säure hervorgegangenes Anion steht;
2) mindestens ein Monomer der Formel (II)
R₆-CH= CR₇-COOH (II)
worin R₆ und R₇ gleich oder verschieden sind und für ein Wasserstoffatom oder einen Methylrest stehen;
und
3) mindestens ein Monomer der Formel (III)
R₆ - CH=CR₇-COXR₈ (III)
worin R₆ und R₇ gleich oder verschieden sind und für ein Wasserstoffatom oder einen Methylrest stehen, X für ein Sauerstoff- oder Stickstoffatom steht und R₈ für einen linearen oder verzweigten Alkylrest mit 1 bis 30 Kohlenstoffatomen steht; umfassen, wobei mindestens eines der Monomere der Formel (Ia), (Ib) oder (III) mindestens eine Fettkette enthält.

39. Zusammensetzung nach Anspruch 38, **dadurch gekennzeichnet, dass** die Monomere der Formel (Ia) und (Ib) aus der Gruppe bestehend aus gegebenenfalls quaternisiertem Dimethylaminoethylmethacrylat, Dimethylaminoethylacrylat, Diethylaminoethylmethacrylat, Diethylaminoethylacrylat, Dimethylaminopropylmethacrylat, Dimethylaminopropylacrylat, Dimethylaminopropylmethacrylamid und Dimethylaminopropylacrylamid ausgewählt sind.

40. Zusammensetzung nach Anspruch 38 oder 39, **dadurch gekennzeichnet, dass** das Monomer der Formel (Ia) unter Acrylamidopropyltrimethylammoniumchlorid und Methacrylamidopropyltrimethylammoniumchlorid ausgewählt ist.

41. Zusammensetzung nach Anspruch 38, **dadurch gekennzeichnet, dass** das Monomer der Formel (II) aus der Gruppe bestehend aus Acrylsäure, Methacrylsäure, Crotonsäure und 2-Methylcrotonsäure ausgewählt ist.

42. Zusammensetzung nach Anspruch 38, **dadurch gekennzeichnet, dass** das Monomer der Formel (III) aus der Gruppe bestehend aus C₁₂-C₂₂-Alkylacrylaten oder -methacrylaten und vorzugsweise C₁₆-C₁₈-Alkylacrylaten oder -methacrylaten ausgewählt ist.

43. Zusammensetzung nach einem der vorhergehenden Ansprüche, **dadurch gekennzeichnet, dass** das oder die Assoziativpolymere in der Zusammensetzung in Gewichtsanteilen zwischen 0,05 und 10% und vorzugsweise zwishen 0,1 und 5%, bezogen auf das Gesamtgewicht der Zusammensetzung, vorliegt.

44. Zusammensetzung nach einem der Ansprüche 1 bis 20, **dadurch gekennzeichnet, dass** es sich bei dem Assoziativpolymer um ein Fettkettenpolymer vom kationischen Typ handelt.

45. Zusammensetzung nach Anspruch 44, **dadurch gekennzeichnet, dass** das Assoziativpolymer unter kationischen Polyurethanen ausgewählt ist.

46. Zusammensetzung nach einem der vorhergehenden Ansprüche, **dadurch gekennzeichnet, dass** das Gewichtsverhältnis von Amid eines Alkanolamins und einer C₁₄-C₃₀-Fettsäure zu nicht oxyethyleniertem Fettalkohol zwischen 0,1 und 10, vorzugsweise zwischen 0,5 und 5, liegt.

47. Zusammensetzung nach einem der vorhergehenden Ansprüche, **dadurch gekennzeichnet, dass** die Zusammensetzung außerdem mindestens ein amphoteres oder kationisches substantives Polymer, das sich von den in einem der Ansprüche 20 bis 44 definierten unterscheidet, enthält.

48. Zusammensetzung nach Anspruch 47, **dadurch gekennzeichnet, dass** es sich bei dem substantiven Polymer um das Homopolymer von Dimethyldiallylammoniumchlorid handelt.

49. Zusammensetzung nach Anspruch 47, **dadurch gekennzeichnet, dass** es sich bei dem substantiven Polymer um ein Polymer mit Wiederholungseinheiten der nachstehenden Formel (W) handelt:

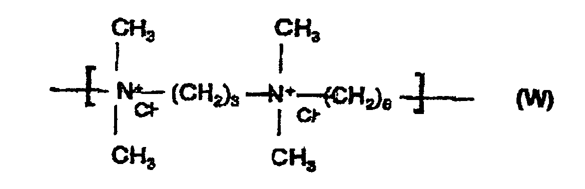

50. Zusammensetzung nach Anspruch 47, **dadurch gekennzeichnet, dass** es sich bei dem substantiven Polymer um ein Polymer mit Wiederholungseinheiten der nachstehenden Formel (U) handelt:

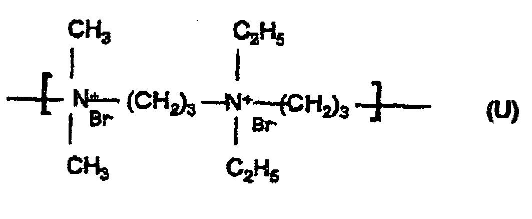

51. Zusammensetzung nach einem der Ansprüche 47 bis 50, **dadurch gekennzeichnet, dass** die kationischen oder amphoteren substantiven Polymere 0,01 bis 10%, vorzugsweise 0,05 bis 5% und noch weiter bevorzugt 0,1 bis 3% des Gesamtgewichts der Zusammensetzung ausmachen.

52. Zusammensetzung nach einem der vorhergehenden Ansprüche, **dadurch gekennzeichnet, dass** sie außerdem mindestens ein Tensid, das unter anionischen, amphoteren, nichtionischen, zwitterionischen und kationischen Tensiden ausgewählt ist, umfasst.

53. Zusammensetzung nach Anspruch 52, **dadurch gekennzeichnet, dass** die Tenside 0,01 bis 40 Gew.-% und vorzugsweise 0,5 bis 30 Gew.-% des Gesamtgewichts der Zusammensetzung ausmachen.

54. Zusammensetzung nach einem der vorhergehenden Ansprüche, **dadurch gekennzeichnet, dass** sie mindestens einen zusätzlichen Verdicker umfasst.

55. Zusammensetzung nach Anspruch 54, **dadurch gekennzeichnet, dass** es sich bei dem zusätzlichen Verdicker um einen Celluloseverdicker, ein Guargummiderivat, ein Gummi mikrobiellen Ursprungs oder einen synthetischen Verdicker handelt.

56. Zusammensetzung nach Anspruch 54 oder 55, **dadurch gekennzeichnet, dass** das oder die zusätzlichen Verdicker 0,01 bis 10 Gew.-% des Gesamtgewichts der Zusammensetzung ausmachen.

57. Zusammensetzung nach einem der vorhergehenden Ansprüche, **dadurch gekennzeichnet, dass** sie außerdem mindestens ein Reduktionsmittel in Mengen von 0,05 bis 1,5 Gew.-%, bezogen auf das Gesamtgewicht der Zusammensetzung, umfasst.

58. Gebrauchsfertige Zusammensetzung nach einem der vorhergehenden Ansprüche, **dadurch gekennzeichnet, dass** sie außerdem ein Oxidationsmittel umfasst.

59. Zusammensetzung nach Anspruch 58, **dadurch gekennzeichnet, dass** das Oxidationsmittel unter Wasserstoffperoxid, Harnstoffperoxid, Alkalimetallbromaten oder -ferricyaniden, Persalzen und Redoxenzymen, gegebenenfalls mit dem jeweiligen Donor oder Cofaktor davon, ausgewählt ist.

60. Zusammensetzung nach Anspruch 59, **dadurch gekennzeichnet, dass** es sich bei dem Oxidationsmittel um Wasserstoffperoxid handelt.

61. Zusammensetzung nach Anspruch 60, **dadurch gekennzeichnet, dass** es sich bei dem Oxidationsmittel um eine wäßrige Wasserstoffperoxidlösung mit einem Titer von 1 bis 40 Volumina handelt.

62. Zusammensetzung nach Anspruch 57, **dadurch gekennzeichnet, dass** sie einen pH-Wert im Bereich von 4 bis 11 besitzt.
